# FASCICULE DE BREVET EUROPEEN

(11) **EP 0 764 149 B1**
(45) Date de publication et mention de la délivrance du brevet: **20.01.1999**
(21) Numéro de dépôt: 95922573.1
(22) Date de dépôt: 07.06.1995
(51) Int. Cl.: C07C 323/59, C07C 323/25, C07C 323/60, A61K 31/195, A61K 31/22

(54) **NOUVEAUX INHIBITEURS DE FARNESYL TRANSFERASE, LEUR PREPARATION ET LES COMPOSITIONS PHARMACEUTIQUES QUI LES CONTIENNENT**
FARNESYL-TRANSFERASE-INHIBITOREN, IHRE HERSTELLUNG UND SIE ENTHALTENDE PHARMAZEUTISCHE ZUSAMMENSETZUNGEN
NOVEL FARNESYL TRANSFERASE INHIBITORS, THEIR PREPARATION AND PHARMACEUTICAL COMPOSITIONS CONTAINING SAME

(30) Priorité: 10.06.1994 FR 9407116; 17.10.1994 FR 9412338
(43) Date de publication de la demande: 26.03.1997
(73) Titulaire: RHONE-POULENC RORER S.A., 92160 Antony (FR)
(72) Inventeur: BAUDOIN, Bernard, F-92370 Chaville (FR); BURNS, Christopher, Rosemont, PA 19010 (US); COMMERCON, Alain, F-94400 Vitry-sur-Seine (FR); GUITTON, Jean-Dominique, F-75013 Paris (FR)
(74) Mandataire: Savina, Jacques
(86) Numéro de dépôt international: FR9500739
(87) Numéro de publication internationale: WO9534535

(56) Documents cités:
- EP-A- 0 461 869
- WO-A-94/00419
- WO-A-94/09766
- JOURNAL OF MEDICINAL CHEMISTRY, vol. 37, no. 6, 18 Mars 1994 WASHINGTON, DC, US, pages 725-732, S.L. GRAHAM, ET AL.: 'Pseudopeptide inhibitors of Ras farnesyl-protein transferase'

## Description

La présente invention concerne de nouveaux inhibiteurs de farnésyl transférase de formule générale : 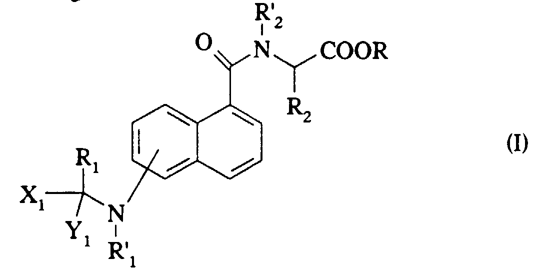 éventuellement leurs sels, leur préparation et les compositions pharmaceutiques qui les contiennent.

L'inhibition de la farnésyl transférase et, par conséquent, de la farnésylation de la protéine Ras, bloque la capacité de la protéine Ras mutée à transformer les cellules normales en cellules cancéreuses.

La séquence C-terminale du gène Ras contient le motif "CAAX" ou "Cys-Aaa₁-Aaa₂-Xaa" dans lequel Aaa représente un aminoacide aliphatique et Xaa représente un aminoacide quelconque.

Il est connu que des tétrapeptides avec une séquence CAAX peuvent inhiber la farnésylation de la protéine Ras. Par exemple, dans la demande PCT WO 91/16340 et dans la demande EP 0 461 869 ont été décrits des peptides inhibiteurs de la farnésyl transférase Cys-Aaa₁-Aaa₂-Xaa qui sont particulièrement représentés par les peptides Cys-Val-Leu-Ser, Cys-Val-Ile-Met et Cys-Val-Val-Met qui manifestent leur activité inhibitrice à des concentrations voisines de 10⁻⁶ ou de 10⁻⁷M.

Il a maintenant été trouvé, et c'est ce qui fait l'objet de la présente invention, que les peptides de formule générale (I) manifestent leur activité inhibitrice (IC₅₀) à des concentrations de l'ordre de 10⁻⁸ ou de 10⁻⁹M.

Dans la formule générale (I),
R₁ représente un radical de formule générale Y-S-A₁- dans lequel Y représente un atome d'hydrogène, ou un reste d'aminoacide ou un reste d'acide gras ou un radical alkyle ou alkoxycarbonyle ou un radical R₄-S- dans lequel R₄ représente un radical alkyle contenant 1 à 4 atomes de carbone éventuellement substitué par un radical phényle ou un radical de formule générale : 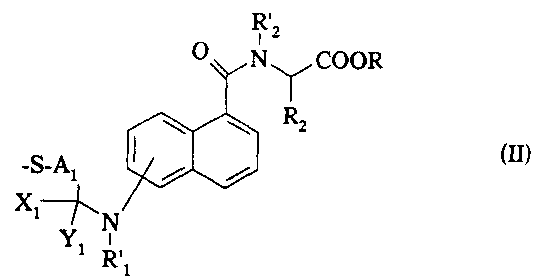 dans laquelle A₁, X₁, Y₁, R'₁, R₂, R'₂ et R sont définis comme ci-après, et A₁ représente un radical alcoylène droit ou ramifié contenant 1 à 4 atomes de carbone éventuellement substitué en α du groupement >C(X₁)(Y₁) par un radical amino, alkylamino contenant 1 à 6 atomes de carbone en chaîne droite ou ramifiée, dialkylamino dont chaque partie alkyle contient 1 à 6 atomes de carbone en chaîne droite ou ramifiée, alcanoylamino contenant 1 à 6 atomes de carbone en chaîne droite ou ramifiée ou alkoxycarbonylamino dont la partie alkyle contient 1 à 6 atomes de carbone en chaîne droite ou ramifiée,
X₁ et Y₁ représentent chacun un atome d'hydrogène ou forment ensemble avec l'atome de carbone auquel ils sont liés un groupement >C=O,
R'₁ représente un atome d'hydrogène ou un radical méthyle,
R₂ représente un radical alkyle, alkényle ou alkynyle droit ou ramifié contenant 1 à 6 atomes de carbone éventuellement substitué par un radical hydroxy, alkoxy contenant 1 à 4 atomes de carbone, mercapto, alkylthio contenant 1 à 4 atomes de carbone, alkylsulfinyle contenant 1 à 4 atomes de carbone ou alkylsulfonyle contenant 1 à 4 atomes de carbone, étant entendu que, lorsque R₂ représente un radical alkyle substitué par un radical hydroxy, R₂ peut former avec la radical carboxy en α une lactone,
R'₂ représente un atome d'hydrogène ou un radical méthyle, et
R représente un atome d'hydrogène ou un radical alkyle contenant 1 à 6 atomes de carbone éventuellement substitué par un radical alkoxy contenant 1 à 4 atomes de carbone, alcoylthio contenant 1 à 4 atomes de carbone, alkylsulfinyle contenant 1 à 4 atomes de carbone, alkylsulfonyle contenant 1 à 4 atomes de carbone, phényle, phénoxy, phénylthio, phénylsulfinyle, phénylsulfonyle, alkylamino contenant 1 à 4 atomes de carbone, dialkylamino dont chaque partie alcoyle contient 1 à 4 atomes de carbone, ou un radical phényle éventuellement substitué par un ou plusieurs atomes ou radicaux choisis parmi les atomes d'halogène et les radicaux alkyles, alkyloxy, alkylthio ou alcanoyle,
étant entendu que le radical 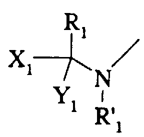 est en position -5 ou -6 du noyau naphtyle.

Plus particulièrement,
R₁ représente un radical de formule Y-S-A₁- dans lequel Y représente un atome d'hydrogène ou un reste lysine ou un reste d'acide gras contenant jusqu'à 20 atomes de carbone et A₁ représente un radical éthylène ou propylène éventuellement substitué par un radical amino ou alkylamino contenant 1 à 4 atomes de carbone,
X₁ et Y₁ représentent chacun un atome d'hydrogène ou forment ensemble avec l'atome de carbone auquel ils sont liés un groupement >C=O,
R'₁ représente un atome d'hydrogène ou un radical méthyle,
R₂ représente un radical alkyle contenant 1 à 4 atomes de carbone éventuellement substitué par un radical hydroxy, méthoxy, mercapto, méthylthio, méthylsulfinyle ou méthylsulfonyle,
R'₂ représente un atome d'hydrogène ou un radical méthyle, et
R représente un atome d'hydrogène ou un radical alcoyle contenant 1 à 4 atomes de carbone, éventuellement substitué par un radical alcoxy, ou un radical phényle.

Plus particulièrement encore,
R₁ représente un radical de formule Y-S-A₁- dans lequel Y représente un atome d'hydrogène et A₁ représente un radical éthylène ou propylène éventuellement substitué par un radical amino,
X₁ et Y₁ représentent chacun un atome d'hydrogène ou forment ensemble avec l'atome de carbone auquel ils sont liés un groupement >C=O,
R'₁ représente un atome d'hydrogène,
R₂ représente un radical méthyle, éthyle, propyle ou butyle éventuellement substitué par un radical hydroxy, méthoxy, mercapto ou méthylthio,
R'₂ représente un atome d'hydrogène, et
R représente un atome d'hydrogène ou un radical alcoyle contenant 1 à 4 atomes de carbone.

Tout particulièrement intéressants sont les produits de formule générale (I) dans laquelle R₁ représente un radical 2-mercapto éthyle ou 1-amino-2-mercapto éthyle, X₁ et Y₁ représentent chacun un atome d'hydrogène ou forment ensemble avec l'atome de carbone auquel ils sont liés un groupement >C=O, R'₁ représente un atome d'hydrogène, R₂ représente un radical n.butyle ou 2-méthylthio éthyle et R'₂ représente un atome d'hydrogène, et R représente un atome d'hydrogène ou un radical méthyle.

La présente invention concerne également les formes stéréoisomères des produits de formule générale (I). Les restes des aminoacides représentés par R₁C(X₁)(Y₁)(NR'₁) et R₃CH(NR'₃)CO-OH ont de préférence la configuration des aminoacides naturels.

La présente invention concerne également les sels minéraux ou organiques des produits de formule générale (I).

Les nouveaux produits selon l'invention peuvent être préparés par application des méthodes connues dérivées des méthodes utilisées plus particulièrement en chimie peptidique pour l'assemblage de chaînes.

Généralement, les produits de formule générale (I), dans laquelle X₁ et Y₁ forment ensemble avec l'atome de carbone auquel ils sont liés un groupement >C=O, sont obtenus à partir de l'acide 5-nitro ou 6-nitro naphtalènecarboxylique-1 sur lequel est condensé un amino acide de formule générale : 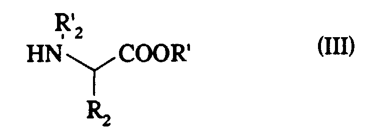 dans laquelle R₂ et R'₂ sont définis comme précédemment et R' représente un radical alkyle contenant 1 à 4 atomes de carbone, éventuellement substitué par un radical phényle, de préférence un radical tert-butyle, en opérant en présence d'un agent de condensation, tel que l'hydroxybenzotriazole et le dicyclohexylcarbodiimide ou le chlorhydrate de N'-(3-diméthylaminopropyl)-N-éthylcarbodiimide, et d'une base, telle que la triéthylamine, dans un solvant organique, tel que le diméthyl-formamide, pour donner un produit de formule générale : 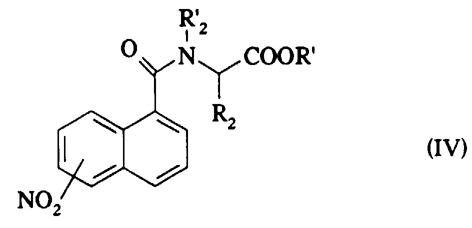 dans laquelle R₂, R'₂ et R' sont définis comme précédemment, qui est réduit, de préférence au moyen de chlorure stanneux ou par l'hydrogène en présence d'un catalyseur tel que le palladium, en produit de formule générale : 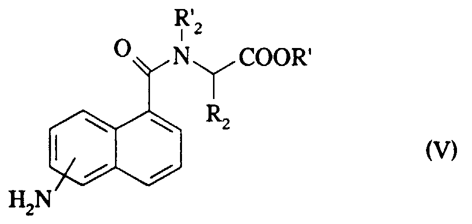 dans laquelle R₂, R'₂ et R' sont définis comme précédemment, sur lequel est condensé un produit de formule générale : 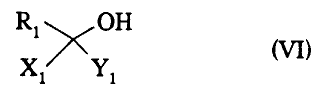 dans laquelle R₁ est défini comme précédemment et X₁ et Y₁ forment ensemble avec l'atome de carbone auquel ils sont liés un groupement >C=O, étant entendu que les fonctions amino et mercapto portées par R₁ sont éventuellement protégées par des groupements protecteurs appropriés tel qu'un radical trityle pour la fonction mercapto ou un radical tert-butoxycarbonyle pour la fonction amino, en opérant de préférence en présence d'un halogénoformiate d'alkyle (chloroformiate d'isobutyle) et d'une base organique (N-méthylmorpholine) dans un solvant organique inerte (tétrahydrofuranne) pour obtenir un produit de formule générale : 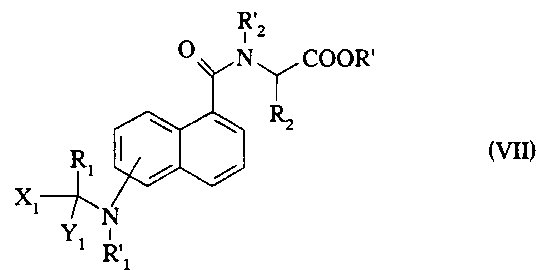 dans laquelle les symboles R₁, R'₁, X₁, Y₁, R₂, R'₂ et R' sont définis comme précédemment dont les groupements protecteurs sont remplacés par des atomes d'hydrogène, au moyen d'acide trifluoroacétique en présence d'éthaneditiol ou de triéthylsilane, lorsque les groupements protecteurs représentent des radicaux trityle, tert-butoxycarbonyle ou tert-butyle, pour obtenir un produit de formule générale (I) dans laquelle X₁ et Y₁ forment ensemble avec l'atome de carbone auquel ils sont liés un groupement >C=O.

Généralement les produits de formule générale (I) dans laquelle les symboles X₁ et Y₁ représentent chacun un atome d'hydrogène peuvent être obtenus par action d'un aldéhyde de formule générale :

R₁-CHO (VIII)

dans laquelle R₁ est défini comme précédemment, étant entendu que les fonctions amino et mercapto portées par R₁ sont éventuellement protégées par des groupements protecteurs appropriés tel qu'un radical trityle pour la fonction mercapto ou un radical tert-butoxycarbonyle pour la fonction amino, sur un produit de formule générale (V) en présence d'un agent réducteur tel que le cyanoborohydrure de sodium, le borohydrure de sodium, le triacétoxyborohydrure de sodium ou l'hydrogène en présence d'un catalyseur. Généralement la réaction s'effectue dans un solvant organique tel qu'un alcool comme le méthanol éventuellement en association avec un autre solvant organique tel qu'un éther comme le tétrahydrofuranne. Il est particulièrement avantageux d'opérer en milieu anhydre.

La condensation de l'aldéhyde avec l'amine étant réalisée, les groupements protecteurs sont remplacés par des atomes d'hydrogène par application des techniques habituelles. Ainsi, les groupements protecteurs Boc ou trityle ou tert-butyle peuvent être remplacés par des atomes d'hydrogène au moyen d'acide trifluoroacétique en présence d'éthanedithiol ou de triéthylsilane.

Lorsque dans la formule générale (I) le symbole R₂ forme avec la fonction carboxy en α une lactone, le traitement en milieu basique du produit correspondant conduit au produit de formule générale (I) dans laquelle R₂ représente un radical alkyle substitué par un radical hydroxy. Généralement, l'ouverture de la lactone s'effectue dès que le pH devient supérieur à 7. Il est particulièrement avantageux d'opérer en présence d'une base minérale (soude, potasse) en milieu hydro-alcoolique tel qu'un mélange eau-méthanol.

Les produits de formule générale (I) dans laquelle R représente un atome d'hydrogène peuvent être obtenus par saponification d'un produit de formule générale (I) dans laquelle R représente un radical alkyle éventuellement substitué ou ou radical phényle éventuellement substitué.

Les produits de formule générale (I) dans laquelle R représente un radical alkyle éventuellement substitué ou un radical phényle éventuellement substitué comme indiqué précédemment peuvent être obtenus par estérification d'un produit de formule générale (I) dans laquelle R représente un atome d'hydrogène dans les conditions habituelles d'estérification qui ne touchent pas au reste de la molécule.

Les acides 5-nitro ou 6-nitro-naphtalène-1-carboxylique peuvent être préparés selon le procédé décrit par T. NAKAYAMA et coll., Chem. Pharm. Bull., 32, 3968 (1984).

Le S-triphénylméthyl-N-tert-butoxycarbonyl-cystéinal peut être préparé selon le procédé décrit dans la demande de brevet européen EP 0 618 221.

Les produits de formule générale (I) peuvent être purifiés selon les méthodes habituelles telles que la chromatographie.

Les exemples suivants illustrent la préparation des produits selon l'invention.

### EXEMPLE 1

On prépare l'acide 5-nitro-naphtalène-1-carboxylique selon la méthode de T. NAKAYAMA et. coll., Chem. Pharm. Bull., 32, 3968 (1984).

A une solution de 2,17 g de l'acide 5-nitro-naphthalène-1-carboxylique dans 30 cm3 de dichlorométhane et 13 cm3 de diméthylformamide, on ajoute 2 g de chlorohydrate de l'ester méthylique de la (L)-méthionine, 1,35 g de 1-hydroxybenzotriazole, 1,4 cm3 de triéthylamine et 2,06 g de dicyclohexylcarbodiimide. Le mélange réactionnel est agité pendant 20 heures à une température voisine de 20°C puis filtré sur verre fritté, lavé par 2 fois 5 cm3 de diméthylformamide et puis concentré à sec sous pression réduite. Le résidu obtenu est dissous dans 100 cm3 de dichlorométhane, lavé par une solution aqueuse d'hydrogénocarbonate de sodium à 10 % (p/v) puis par une solution aqueuse d'acide acétique à 10 % et enfin par une solution aqueuse saturée de chlorure de sodium. La phase organique est séchée sur sulfate de magnésium, filtrée puis concentrée à sec sous pression réduite. On obtient 3,4 g d'un solide marron que l'on purifie par chromatographie sur silice [éluant :dichlorométhane-acétate d'éthyle (95-5 en volumes)]. On obtient ainsi 2,6 g de l'ester méthylique de la N-[(5-nitro-naphthyl)-1-carbonyl]-L-méthionine sous forme d'un solide dont les caractéristiques sont les suivantes :
- spectre de résonance magnétique nucléaire du proton .(300 MHz ; (CD₃)₂SO d6 ; δ en ppm) : de 2,00 à 2,25 (mt, 2H : CH₂); 2,11 (s, 3H : SCH₃); 2,65 (mt, 2H : SCH₂); 3,77 (s, 3H : OCH₃); 4,70 (mt, 1H : CHN) ; 7,82 et 7,89 (2t, J = 8,5 Hz, 2H : H en 3 et H en 7) ; 7,85 (d, J = 8,5 Hz, 1H : H en 2) ; 8,36 (d, J = 8,5 Hz, 1H : H en 4) ; 8,43 (d, J = 8,5 Hz, 1H : H en 8) ; 8,57 (d, J = 8,5 Hz, 1H : H en 6) ; 9,17 (d, J = 7,5 Hz, 1H : CONH).

A une solution de 1,09 g de l'ester méthylique de la N-[(5-nitro-naphthyl)-1-carbonyl]-L-méthionine dans 23 cm3 d'acétate d'éthyle et 6 cm3 d'éthanol, on ajoute 3,39 g de dihydrate de chlorure d'étain (II). Le mélange réactionnel est agité pendant 30 minutes à une temperature voisine de 70°C, versé sur de la glace puis amené à pH voisin de 7-8 par addition d'une solution aqueuse d'hydrogénocarbonate de sodium à 5 %. (p/v). Le mélange obtenu est filtré sur verre fritté garni de célite. La phase organique est séparée par décantation et la phase aqueuse est extraite 3 fois par 50 cm3 d'acétate d'éthyle. Les phases organiques sont réunies, séchées sur du sulfate de magnésium, filtrées et concentrées à sec sous pression réduite. On obtient ainsi 0,9 g d'ester méthylique de la N-[(5-amino-naphthyl)-1-carbonyl]-L-méthionine sous forme d'une huile dont les caractéristiques sont les suivantes :
- spectre de résonance magnétique nucléaire du proton (200 MHz, (CD₃)₂SO d6 avec addition de quelques gouttes de CD₃COOD d4, δ en ppm) : de 1,95 à 2,25 (mt, 2H : CH₂); 2,09 (s, 3H : SCH₃); 2,62 (mt, 2H : SCH₂); 3,71 (s, 3H : OCH₃); 4,63 (mt, 1H : CHCOO) ; 6,71 (d, J = 8,5 Hz, 1H : H en 6) ; de 7,15 à 7,60 (mt, 4H : H aromatiques) ; 8,18 (d, J = 8,5 Hz, 1H : H en 8) ; 8,84 (d résiduel, J = 7,5 Hz, 1H : CONH).

A une solution de 1,25 g de N-tert-butoxycarbonylamino-S-triphénylméthyl-L cystéine et de 0,3 cm3 de N-méthylmorpholine dans 25 cm3 de tétrahydrofuranne, à une température voisine de -15°C, on ajoute 0,35 cm3 de chloroformiate d'isobutyle puis une solution de 0,9 g de l'ester méthylique de la N-[(5-amino-naphthyl)-1-carbonyl]-L-méthionine dans 20 cm3 de tétrahydrofuranne. Le mélange réactionnel est agité pendant 2 jours à une température voisine de 20°C, puis filtré et concentré à sec sous pression réduite. Le résidu obtenu est dissous dans 50 cm3 d'acétate d'éthyle, lavé par de l'eau distillée, puis par une solution aqueuse 0,5N d'hydrogénocarbonate de sodium, une solution aqueuse d'acide citrique à 10 % (p/v), et enfin par de l'eau distillée. La phase organique est séchée sur du sulfate de magnésium, filtrée et concentrée à sec sous pression réduite. On obtient ainsi 2 g d'une meringue jaune que l'on purifie par chromatographie sur silice [éluant : dichlorométhane-acétate d'éthyle (90-10 en volumes)]. On obtient 1,5 g de l'ester méthylique de la N-[5-(2(R)-tert-butoxycarbonylamino-3-triphénylméthylthio-propionylamino)-naphthyl-1-carbonyl]-L-méthionine sous forme d'un solide dont les caractéristiques sont les suivantes :
- spectre de résonance magnétique nucléaire du proton (300 MHz, (CD₃)₂SO d6, δ en ppm) : 1,43 (s, 9H : C(CH₃)₃) ; de 2,00 à 2,25 (mt, 4H : CH₂ et CH₂SAr) ; 2,08 (s, 3H : SCH₃); 2,60 (mt, 2H : SCH₂); 3,74 (s, 3H : OCH₃); 4,32 (mt, 1H : NCH) ; 4,64 (mt, 1H : CHCOO) ; de 7,20 à 7,80 (mt, 20H : H aromatiques et CONH) ; de 8,00 à 8,15 (mt, 2H : H en 8 et H en 6) ; 9,02 (d, J = 8 Hz, 1H : CONH) ; 10,08 (s, 1H : ArNH).

A une solution de 0,45 g de l'ester méthylique de la N-[5-(2(R)-tert-butoxycarbonylamino-3-triphénylméthylthio-propionylamino)-naphthyl-1-carbonyl]-L-méthionine dans 1 cm3 d'eau et 10 cm3 de tétrahydrofuranne, à une température voisine de 5°C, on ajoute 0,061 g de lithine dihydratée. La solution est agitée pendant 4 heures à une température voisine de 20°C et puis concentrée sous pression réduite. On obtient 0,47 g de N-[5-(2(R)-tert-butoxycarbonylamino-3-triphénylméthylthiopropionylamino)-naphthyl-1-carbonyl]-L-méthionine sous forme d'une meringue.

A un mélange de 0,44 g de N-[5-(2(R)-tert-butoxycarbonylamino-3-triphénylméthylthio-propionylamino)-naphthyl-1-carbonyl]-L-méthionine dans 2 cm3 d'eau et 2 cm3 d'éthanedithiol, à une température voisine de 5°C, on ajoute 2 cm3 d'acide trifluoroacétique. Le mélange réactionnel est ensuite agité pendant 2 heures à une température voisine de 20°C, puis on ajoute, goutte à goutte, 20 cm3 d'acide trifluoroacétique. Le mélange réactionnel est agité pendant 2 heures puis filtré et concentré sous pression réduite. Le résidu obtenu est trituré 2 fois par 30 cm3 d'éther éthylique puis séché sous pression réduite. On obtient ainsi 0,23 g du trifluoroacétate de la N-[5-(2(R)-amino-3-mercapto-propionylamino)-naphthyl-1-carbonyl}-L-méthionine sous forme d'un solide blanc dont les caractéristiques sont les suivantes :
- spectre de résonance magnétique nucléaire du proton (200 MHz, (CD₃)₂SO d6, δ en ppm) : de 1,90 à 2,25 (mt, 2H : CH₂); 2,10 (s, 3H : SCH₃); 2,65 (mt, 2H : SCH₂) ; 3,15 (d, J = 7 Hz, 2H : SCH₂) ; 4,28 (mt, 1H : NCH) ; 4,61 (mt, 1H : CHCOO) ; de 7,45 à 7,80 (mt, 4H : H aromatiques) ; de 8,10 à 8,30 (mt, 2H : H en 8 et H en 6) ; 8,90 (d, J = 8 Hz, 1H : CONH) ; 10,20 (mf, 1H : ArNHCO)
- analyse élémentaire : C₁₉H₂₃N₃O₄S₂, 1,5 CF₃CO₂H :

| | | | | |
|---|---|---|---|---|
| Calculé (%) | C = 45,78 ; | H = 4,33 ; | N = 7,45 ; | S = 11,37 |
| Trouvé (%) | C = 45,92 ; | H = 4,05 ; | N = 7,54 ; | S = 11,66. |

### EXEMPLE 2

En opérant comme dans l'exemple 1 pour la préparation du trifluoroacétate de la N-[5-(2(R)-amino-3-mercapto-propionylamino)-naphtyl-1-carbonyl]-L-méthionine, mais à partir de 0,52 g d'ester méthylique de la N-[5-(2(R)-tert-butoxycarbonylamino-3-triphénylméthylthio-propionylamino)-naphtyl-1-carbonyl]-L-méthionine, on obtient 0,17 g de trifluoroacétate de l'ester méthylique de la N-[5-(2(R)-amino-3-mercapto-propionylamino)-naphtyl-1-carbonyl]-L-méthionine dont les caractéristiques sont les suivantes :
- spectre de résonance magnétique nucléaire du proton (300 MHz, (CD₃)₂SO d6, δ en ppm) : 2,08 (mt, 2H : CH₂); 2,10 (s, 3H : SCH₃); 2,64 (mt, 2H : CH₂S); 3,21 (d, J = 6 Hz, 2H : SCH₂) ; 3,78 (s, 3H : COOCH₃) ; 4,39 (t, J = 6 Hz, 1H : CHN) ; 4,69 (mt, 1H: CHCOO) ; de 7,60 à 7,80 (mt, 4H : H 2 - H 3 - H 6 et H 7) ; 8,13 (d, J = 8,5 Hz, 1H : H 8) ; 8,25 (dd, J = 7,5 et 2,5 Hz, 1H : H 4) ; de 8,00 à 8,60 (mf, 2H : NH₂) ; 9,05 (d, J = 7.5 Hz, 1H : ArCONH) ; 10,60 (mf, 1H : ArNHCO).
- analyse élémentaire : C₂₀H₂₅N₃O₄S₂, 1,33 CF₃CO₂H :

| | | | | |
|---|---|---|---|---|
| Calculé (%) | C = 46,33 ; | H = 4,52 ; | N = 7,15 ; | S = 10,91 |
| Trouvé (%) | C = 46,2 ; | H = 4,5 ; | N = 7,3 ; | S = 11,4 |

### EXEMPLE 3

A une solution de 0,55 g d'ester méthylique de la N-[(5-amino-naphthyl)-1 carbonyl]-L-méthionine dans 30 cm3 de méthanol on ajoute 0,74 g de S-triphénylméthyl-N-tert-butoxycarbonyl-cystéinal préparé selon le procédé décrit dans la demande de brevet européen EP 0 618 221, 0,1 cm3 d'acide acétique concentré, et du tamis moléculaires (3Å). Le mélange réactionnel est agité pendant 20 heures à une température voisine de 20°C puis on ajoute 0,32 g de cyanoborohydrure de sodium. Le mélange réactionnel est agité pendant 24 heures à une température voisine de 20°C, puis filtré sur verre fritté garni de célite. Le verre fritté est lavé par du méthanol. Le filtrat, concentré sous pression réduite, donne une pâte qui est purifiée par chromatographie sur silice [éluant : dichlorométhane-acétate d'éthyle (90-10 en volumes)]. On obtient ainsi 0,3 g de l'ester méthylique de la N-[5-(2(R)-tert-butoxycarbonylamino-3-triphénylméthylthio-propylamino)-naphthyl-1-carbonyl]-L-méthionine sous forme d'un solide jaune dont les caractéristiques sont les suivantes :
- spectre de résonance magnétique nucléaire du proton : (300 MHz, (CD₃)₂SO d6, δ en ppm) : 1,42 (s, 9H : C(CH₃)₃) ; 2,08 (mt, 2H : CH₂); 2,10 (s, 3H : SCH₃); de 2,25 à 2,55 (mt, 2H : CH₂S); 2,65 (mt, 2H : CH₂S); de 3,00 à 3,25 (mt, 2H : NCH₂); 3,75 (s, 3H : COOCH₃) ; 3,82 (mt, 1H : NCH) ; 4,76 (mt, 1H : CHCOO) ; 6,15 (mf, 1H : ArNH) ; 6,52 (d large, J = 7,5 Hz, 1H : H aromatique en ortho de l'amine) ; 7,02 (d, J = 8 Hz, 1H : OCONH) ; de 7,10 à 7,60 (mt, 19H : H aromatiques) ; 8,02 (d large, J = 8 Hz, 1H : H aromatique en ortho de l'amide) ; 8,92 (d, J = 7,5 Hz, 1H : CONH).

A une solution de 0,3 g de l'ester méthylique de la N-[5-(2(R)-tert-butoxycarbonylamino-3-triphénylméthylthio-propylamino)-naphthyl-1carbonyl]-L-méthionine dans 1 cm3 d'eau et 10 cm3 de tétrahydrofuranne, à une température voisine de 5°C, on ajoute 0,04 g de lithine dihydratée. La solution est agitée pendant 20 heures à une température voisine de 20°C puis concentrée sous pression réduite. On obtient ainsi 0,3 g de N-[5-(2(R)-tert-butoxycarbonylamino-3-triphénylméthylthiopropylamino)-naphthyl-1-carbonyl]-L-méthionine sous forme d'une meringue.

A un mélange de 0,3 g de N-[5-(2(R) -tert-butoxycarbonylamino-3-triphénylméthylthio-propylamino)-naphthyl-1-carbonyl}-L-méthionine dans 1,5 cm3 d'eau et 1,5 cm3 d'éthanedithiol, on ajoute, à une température voisine de 5°C, 1,5 cm3 d'acide trifluoroacétique. Le mélange réactionnel est agité pendant 2 heures à une température voisine de 20°C, puis on ajoute, goutte à goutte, 15 cm3 d'acide trifluoroacétique. Le mélange réactionnel est agité pendant 3 heures puis concentré sous pression réduite. Le résidu obtenu est trituré 3 fois par 25 cm3 d'éther éthylique puis séché sous pression réduite. On obtient ainsi 0,14 g de trifluoroacétate de N-[5-(2(R)-amino-3-mercapto-propylamino)-naphthyl-1-carbonyl]-L-méthionine sous forme d'une pâte jaune dont les caractéristiques sont les suivantes :
- spectre de résonance magnétique nucléaire du proton (200 MHz, (CD₃)₂SO d6 avec addition de quelques gouttes de CD₃COOD d4, δ en ppm) : de 1,95 à 2,20 (mt, 2H : CH₂); 2,10 (s, 3H : SCH₃); de 2,50 à 2,70 (mt, 2H : CH₂S); 2,88 (mt, 2H : CH₂S); de 3,30 à 3,70 (mt, 3H : NCH₂ et NCH) ; 4,59 (mt, 1H : CHCOO) ; 6,30 (mf: ArNH résiduaire) ; 6,71 (d large, J = 8 Hz, 1H : H aromatique en ortho de l'amine) ; de 7,30 à 7,60 (mt, 4H : H aromatiques) ; 8,26 (d large, J = 8,5 Hz, 1H : H aromatique en ortho de l'amide) ; 8,70 (d, J = 9 Hz, 1H : CONH résiduaire).
- analyse élémentaire : C₁₉H₂₅N₃O₃S₂, 1,25 CF₃CO₂H

| | | | | |
|---|---|---|---|---|
| Calculé (%) | C = 46,94 ; | H = 4,81 ; | N = 7,64 ; | S = 11,66 |
| Trouvé (%) | C = 46,69 ; | H = 4,32 ; | N = 7,46 ; | S = 11,90. |

### EXEMPLE 4

En opérant comme dans l'exemple 3 pour la préparation du trifluoroacétate de la N-[5-(2(R)-amino-3-mercapto-propylamino)-naphtyl-1-carbonyl]-L-méthionine, mais à partir de 0,33 g d'ester méthylique de la N-[5-(2(R)-tert-butoxycarbonylamino-3-triphénylméthylthio-propylamino)-naphtyl-1-carbonyl]-L-méthionine, on obtient 0,05 g de trifluoroacétate de l'ester méthylique de la N-[5-(2(R)-amino-3-mercaptopropylamino)-naphtyl-1-carbonyl]-L-méthionine dont les caractéristiques sont les suivantes :
- spectre de résonance magnétique nucléaire du proton (300 MHz, (CD₃)₂SO d6, δ en ppm) : 2,10 (mt, 2H : CH₂); 2,12 (s, 3H : SCH₃); 2,63 (mt, 2H : CH₂S); 2,92 (mt, 2H : CH₂S); de 3,30 à 3,70 (mt : NCH₂CHN) ; 3,76 (s, 3H : COOCH₃) ; 4,79 (mt, 1H : CHCOO) ; 6,40 (mf, 1H : ArNH) ; 6,72 (d, J = 8 Hz, 1H : H 6) ; 7,40 (t, J = 8 Hz, 1H : H 7) ; 7,52 (d, J = 8 Hz, 1H : H 8) ; de 7,40 à 7,60 (mt, 2H : H 2 et H 3) ; 8,13 (mf, 3H : NH₃⁺CF₃COO⁻); 8,30 (d large, J = 8 Hz, 1H : H 4) ; 8,92 (d, J = 7,5 Hz, 1H : ArCONH).
- analyse élémentaire : C₂₀H₂₇N₃O₃S₂, 1,2 CF₃CO₂H :

| | | | | |
|---|---|---|---|---|
| Calculé (%) | C = 48,18 ; | H = 5,09 ; | N = 7,52 ; | S = 11,48 |
| Trouvé (%) | C = 48,0 ; | H = 5,1 ; | N = 7,7 ; | S = 11,9 |

### EXEMPLE 5

En opérant comme dans l'exemple 1 pour la préparation de l'ester méthylique de N-[(5-nitro-naphthyl)-1-carbonyl]-L-méthionine, mais à partir de 1,1 g d'acide 5-nitro-naphthalène-1-carboxylique et de l'ester méthylique de la L-norleucine, on obtient 1,3 g d'ester méthylique de la N-[(5-nitro-naphthyl)-1-carbonyl]-L-norleucine.

En opérant comme dans l'exemple 1 pour la préparation de l'ester méthylique de N-[(5-amino-naphthyl)-1-carbonyl]-L-méthionine, mais à partir de 1,3 g d'ester méthylique de N-[(5-nitro-naphthyl)-1-carbonyl]-L-norleucine, on obtient 1,2 g d'ester méthylique de N-[(5-amino-naphthyl)-1-carbonyl]-L-norleucine.

En opérant comme dans l'exemple 3 pour la préparation de l'ester méthylique de la N-[5-(2(R)-tert-butoxycarbonylamino-3-triphénylméthylthio-propylamino)naphthyl-1-carbonyl]-L-méthionine, mais à partir de 1,4 g d'ester méthylique de N-[(5-amino-naphthyl)-1-carbonyl]-L-norleucine, on obtient 0,67 g d'ester méthylique de la N-[5-(2(R)-tert-butoxycarbonylamino-3-triphénylméthylthio-propylamino)naphthyl-1-carbonyl]-L-norleucine dont les caractéristiques sont les suivantes :
- spectre de résonance magnétique nucléaire du proton (200 MHz, (CD₃)₂SO d6, δ en ppm) : 0,91 (t, J = 7,5 Hz, 3H : CH₃); de 1,25 à 1,60 (mt, 4H : CH₂); 1,43 (s, 9H : C(CH₃)₃) ; 1,80 (mt, 2H : CH₂); de 2,20 à 2,70 (mt, 2H : CH₂S); de 3,00 à 3,50 (mt, 2H : NCH₂) ; 3,75 (s, 3H : COOCH₃) ; 3,82 (mt, 1H : NCH) ; 4,50 (mt, 1H : CHCOO) ; 6,10 (mf, 1H : ArNH) ; 6,52 (d large, J = 7,5 Hz, 1H : H aromatique en ortho de l'amine); 6,97 (d, J = 8,5 Hz, 1H : OCONH) ; de 7,00 à 7,60 (mt, 19H : H aromatiques) ; 8,05 (d large, J = 8 Hz, 1H : H aromatique en ortho de l'amide) ; 8,80 (d, J = 7 Hz, 1H : CONH).
- spectre de masse (DIC) : M/Z = 746 (MH⁺)

En opérant comme dans l'exemple 3 pour la préparation de la N-[5-(2(R)-tert-butoxycarbonylamino-3-triphénylméthylthio-propylamino)-naphthyl-1-carbonyl}-L-méthionine, mais à partir de 0,65 g d'ester méthylique de la N-[5-(2(R)-tert-butoxy-carbonylamino-3-triphénylméthylthio-propylamino)-naphthyl-1-carbonyl]-L-norleucine, on obtient 0,6 g de la N-[5-(2(R)-tert-butoxycarbonylamino-3-triphénylméthylthio-propylamino)-naphthyl-1-carbonyl]-L-norleucine.

En opérant comme dans l'exemple 3 pour la préparation du trifluoroacétate de la N-[5-(2(R)-amino-3-mercapto-propylamino)-naphthyl-1-carbonyl]-L-méthionine, mais à partir de 0,6 g de la N-[5-(2(R)-tert-butoxycarbonylamino-3-triphénylméthylthio-propylamino)-naphthyl-1-carbonyl}-L-norleucine, on obtient 0,06 g de trifluoroacetate de la N-[5-(2(R)-amino-3-mercapto-propylamino)-naphthyl-1 carbonyl]-L-norleucine dont les caractéristiques sont les suivantes :
- spectre de résonance magnétique nucléaire du proton (250 MHz, (CD₃)₂SO d6, δ en ppm) : 0,93 (t, J =7,5 Hz, 3H : CH₃); 1,40 (mt, 4H : CH₂) ; 1,81 (mt, 2H : CH₂); 2,91 (mt, 2H : CH₂S); de 3,30 à 3,70 (mt, 3H : CHN et NCH₂); 4,44 (mt, 1H : CHCOO) ; 6,40 (mf, 1H : ArNH) ; 6,71 (d large, J = 7,5 Hz, 1H : H aromatique en ortho de l'amine) ; de 7,30 à 7,60 (mt, 4H : H aromatiques) ; 8,10(mf, 2H,: NH₂) ; 8,30 (d large, J = 8 Hz, 1H : H aromatique en ortho de l'amide) ; 8,73 (d, J = 7,5 Hz, 1H : CONH)
- analyse élémentaire : C₂₀H₂₇N₃O₃S, CF₃CO₂H

| | | | | |
|---|---|---|---|---|
| Calculé (%) | C = 52,48 ; | H = 5,60 ; | N = 8,34 ; | S = 6,37 |
| Trouvé (%) | C = 51,19 ; | H = 5,46 ; | N = 7,93 ; | S = 6,18 |

- spectre de masse (LSIMS) : M/Z = 390 (MH⁺).

### EXEMPLE 6

En opérant comme dans l'exemple 1 pour la préparation l'ester méthylique de la N-[(5-nitro-naphtyl)-1-carbonyl]-L-méthionine mais à partir de 4,7 g d'ester éthylique de la L-méthionine, on obtient 7,35 g d'ester éthylique de la N-[(5-nitro-naphtyl)-1-carbonyl]-L-méthionine dont les caractéristiques sont les suivantes :
- spectre de résonance magnétique nucléaire du proton (300 MHz, (CD₃)₂SO d6, δ en ppm) : 1,28 (t, J = 7,5 Hz, 3H : CH₃ de l'éthyle) ; 2,08 (mt, 2H : CH₂) ; 2,11 (s, 3H : SCH₃); 2,65 (mt, 2H : CH₂S) ; 4,22 ( mt, 2H : COOCH₂ de l'éthyle) ; 4,66 (mt, 1H : CHCOO) ; de 7,80 à 8,00 (mt, 3H : H 2 - H 3 et H 7) ; 8,36 (d, J = 7,5 Hz, 1H : H 4) ; 8,46 et 8,56 (2 d, J = 8 Hz, 1H : H 6 et H 8) ; 9,17 (d, J = 7,5 Hz, 1H : ArCONH).

En opérant comme dans l'exemple 1 pour la préparation de l'ester méthylique de la N-[(5-amino-naphtyl)-1-carbonyl]-L-méthionine mais à partir de 1,38 g d'ester éthylique de la N-[(5-nitro-naphtyl)-1-carbonyl]-L-méthionine, on obtient 1,02 g d'ester éthylique de la N-[(5-amino-naphtyl)-1-carbonyl]-L-méthionine qui sont utilisés dans l'étape suivante sans autre purification.

En opérant comme dans l'exemple 3 pour la préparation de l'ester méthylique de la N-[5-(2(R)-tert-butoxycarbonylamino-3-triphénylméthylthio-propylamino)-naphtyl-1-carbonyl]-L-méthionine mais à partir de 1 g d'ester éthylique de la N-[(5-amino-naphtyl)-1-carbonyl]-L-méthionine, on obtient 0,94 g d'ester éthylique de la N-[5-(2(R)-tert-butoxycarbonylamino-3-triphénylméthylthio-propylamino)-naphtyl-1-carbonyl]-L-méthionine qui sont utilisés dans l'étape suivante sans autre purification.

En opérant comme dans l'exemple 3 pour la préparation du trifluoroacétate de la N-[5-(2(R)-amino-3-mercapto-propylamino)-naphtyl-1-carbonyl]-L-méthionine mais à partir de 0,4 g d'ester éthylique de la N-[5-(2(R)-tert-butoxycarbonylamino-3-triphénylméthylthio-propylamino)-naphtyl-1-carbonyl]-L-méthionine, on obtient 0,033 g de l'ester éthylique de la N-[5-(2(R)-amino-3-mercapto-propylamino)-naphtyl-1-carbonyl]-L-méthionine sous forme d'une poudre dont les caractéristiques sont les suivantes:
- spectre de résonance magnétique nucléaire du proton (400 MHz, (CD₃)₂SO d6, δ en ppm) : 1,30 (t, J = 7,5 Hz, 3H : CH₃ de l'éthyle) ; 2,10 (mt, 2H : CH₂) ; 2,11 (s, 3H : SCH₃); 2,65 (mt, 2H : CH₂S) ; 2,90 (AB dédoublé, J = 14 et 5 Hz, 2H : CH₂S) ; de 3,30 à 3,70 (mt : NCH₂CHN) ; 4,21 (mt, 2H : COO CH₂ de l'éthyle) ; 4,65 (mt, 1H : CHCOO) ; 6,38 (mt, 1H : ArNH) ; 6,72 (d, J = 8 Hz, 1H : H 6); 7,40 (t, J = 8 Hz, 1H : H 7) ; 7,52 (d, J = 8 Hz, 1H : H 8) ; de 7,40 à 7,60 (mt, 2H : H 2 et H 3) ; 8,30 (d large, J = 8 Hz, 1H : H 4) ; 8,90 (d, J = 7,5 Hz, 1H : ArCONH).
- analyse élémentaire : C₂₁H₂₉N₃O₃S₂, CF₃CO₂H :

| | | | | |
|---|---|---|---|---|
| Calculé (%) | C = 50,26 ; | H = 5,5 ; | N = 7,65 ; | S = 11,67 |
| Trouvé (%) | C = 49,9 ; | H = 5,8 ; | N = 7,7 ; | S = 11,7 |

### EXEMPLE 7

A une solution de 5,36 g N-tert-butoxycarbonyl-L-méthionine dans 100 cm3 de diéthyléther on ajoute 2,68 g de 5-indanol, puis 0,24 g de 4-diméthylaminopipéridine et 4,96 g de 1,3-dicyclohexylcarbodiimide. Le mélange réactionnel est agité à une température voisine de 20°C jusqu'à estérification complète, puis filtré sur un verre fritté et lavé successivement par trois fois 100 cm3 d'eau, puis par 100 cm3 d'une solution aqueuse d'acide acétique à 5 % (v/v), puis par 100 cm3 d'eau et enfin par deux fois 100 cm3 d'une solution aqueuse d'hydrogénocarbonate de sodium à 10 % (p/v). La phase organique est séchée sur du sulfate de magnésium, filtrée et concentrée à sec sous pression réduite. On obtient 4,03 g de l'ester de 5-indanyle de la N-tert-butoxycarbonyl-L-méthionine qui sont utilisés dans l'étape suivante sans autre purification.

A une solution de 0,96 g de l'ester de 5-indanyle de la N-tert-butoxycarbonyl-L-méthionine dans 25 cm3 de dichlorométhane on ajoute 5 cm3 d'acide trifluoroacétique. Le mélange réactionnel est agité à une température voisine de 20°C pendant 3 heures. On obtient, après élimination du solvant et cristallisation dans le diisopropyléther, 0,5 g de l'ester de 5-indanyle de la L-méthionine qui sont utilisés dans l'étape suivante sans autre purification.

En opérant comme dans l'exemple 1 pour la préparation de l'ester méthylique de la N-[(5-nitro-naphtyl)-1-carbonyl]-L-méthionine, mais à partir 0,5 g de l'ester de 5-indanyle de la L-méthionine, on obtient 0,28 g de l'ester de 5-indanyle de la N-[(5-nitro-naphtyl)-1-carbonyl]-L-méthionine dont les caractéristiques sont les suivantes :
- spectre de résonance magnétique nucléaire du proton (250 MHz, (CD₃)₂SO d6, δ en ppm) : 2,10 (mt, 2H : CH₂ de l'indanyle) ; 2,14 (s, 3H : SCH₃) ; 2,24 (mt, 2H : CH₂) ; 2,73 (mt, 2H : CH₂S) ; 2,87 et 2,90 (2 t, J = 7,5 Hz, 2H chacun : ArCH₂ de l'indanyle) ; 4,88 (mt, 1H : CHCOO) ; 6,95 (dd, J = 9 et 2 Hz, 1H : H 6 de l'indanyle) ; 7,06 (d, J = 2 Hz, 1H : H 4 de l'indanyle) ; 7,30 (d, J = 9 Hz, 1H : H 7 de l'indanyle) ; 7,76 (t, J = 8 Hz, 1H : H 7) ; de 7,85 à 8,00 (mt, 2H : H 2 et H 3) ; 8,35 et 8,60 (2 d, J = 8 Hz, 1H chacun : H 6 et H 8) ; 8,43 (dd, = 7,5 et 3 Hz, 1H : H 4) ; 9,36 (d, J = 7,5 Hz, 1H : ArCONH).

En opérant comme dans l'exemple 1 pour la préparation de l'ester méthylique de la N-[(5-amino-naphtyl)-1-carbonyl]-L-méthionine, mais à partir de 4,64 g de l'ester de 5-indanyle de la N-[(5-nitro-naphtyl)-1-carbonyl]-L-méthionine, on obtient 3,40 g de l'ester de 5-indanyle de la N-[(5-amino-naphtyl)-1-carbonyl]-L-méthionine dont les caractéristiques sont les suivantes :
- spectre de résonance magnétique nucléaire du proton (200 MHz, (CD₃)₂SO d6, δ en ppm) : 2,07 (mt, 2H : CH₂ de l'indanyle) ; 2,14 (s, 3H : SCH₃) ; de 2,10 à 2,30 (mt, 2H : CH₂) ; 2,72 (mt, 2H : CH₂S) ; 2,88 et 2,94 (2 t, J = 7 Hz, 2H chacun : ArCH₂ de l'indanyle) ; 4,82 (mt, 1H : CHCOO) ; 5,81 (s large, 2H : NH₂) ; 6,72 (d large , J = 7,5 Hz, 1H : H 6) ; 6,94 (dd, J = 8 et 2 Hz, 1H : H 6 de l'indanyle) ; 7,05 (d, = 2 Hz, 1H : H 4 de l'indanyle) ; 7,22 (t, J = 7,5 Hz, 1H : H 7) ; 7,30 (d, J = 8 Hz, 1H : H 7 de l'indanyle) ; de 7,35 à 7,55 (mt, 3H : H 2 - H 3 et H 8) ; 8,20 (d large, J = 8 Hz, 1H : H 4) ; 9,08 (d, J = 7,5 Hz, 1H : ArCONH).

En opérant comme dans l'exemple 3 pour la préparation de l'ester méthylique de la N-[5-(2(R)-tert-butoxycarbonylamino-3-triphénylméthylthiopropylamino)-naphtyl-1-carbonyl]-L-méthionine mais à partir 0,22 g de l'ester de 5-indanyle de la N-[(5-amino-naphtyl)-1-carbonyl]-L-méthionine, on obtient 0,38 g de l'ester de 5-indanyle de la N-[5-(2(R)-tert-butoxycarbonylamino-3-triphénylméthylthio-propylamino)-naphtyl-1-carbonyl]-L-méthionine dont les caractéristiques sont les suivantes :
- spectre de résonance magnétique nucléaire du proton (250 MHz, (CD₃)₂SO d6 avec ajout de quelques gouttes de CD₃COOD d4, δ en ppm) : 1,38 (s, 9H : OC(CH₃)₃) ; 2,07 (mt, 2H : CH₂ de l'indanyle) ; 2,12 (s, 3H : SCH₃) ; 2,25 (mt, 2H : CH₂) ; de 2,55 à 2,80 (mt, 4H : CH₂S) ; 2,86 et 2,90 (2 t, J = 7,5 Hz, 2H chacun : ArCH₂ de l'indanyle) ; de 3,00 à 3,30 (mt, 2H : NCH₂) ; 3,81 (mt, 1H : CHN) ; 4,82 (mt, 1H : CHCOO) ; 6,52 (d large, J = 8 Hz, 1H : H 6) ; 6,92 (dd, J = 8 et 2 Hz, 1H : H6 de l'indanyle) ; 7,02 (d, J = 2 Hz, 1H : H 4 de l'indanyle) ; de 7,10 à 7,60 (mt, 20H : H 7 de l'indanyle - SC(C₆H₅)₃ - H 2 - H 3 - H 7 et H 8) ; 8,03 (d large, J = 8,5 Hz, 1H : H 4).

En opérant comme dans l'exemple 3 pour la préparation du trifluoroacétate de la N-[5-(2(R)-amino-3-mercapto-propylamino)-naphtyl-1-carbonyl]-L-méthionine mais à partir de 0,38 g de l'ester de 5-indanyle de la N-[5-(2(R)-tert-butoxycarbonylamino-3-triphénylméthylthio-propylamino)-naphtyl-1-carbonyl]-L-méthionine, on obtient 0,064 g du trifluoroacétate de l'ester de 5-indanyle de la N-[5-(2(R)-amino-3-mercapto-propylamino)-naphtyl-1-carbonyl]-L-méthionine dont les caractéristiques sont les suivantes :
- spectre de résonance magnétique nucléaire du proton (300 MHz, (CD₃)₂SO d6, δ en ppm) : 2,06 (mt, 2H : CH₂ de l'indanyle) ; 2,12 (s, 3H : SCH₃) ; 2,22 (mt, 2H : CH₂) ; 2,70 (mt, 2H : CH₂S) ; de 2,75 et 2,85 (mt, 6H chacun : ArCH₂ de l'indanyle et CH₂S) ; de 3,00 à 3,60 (mt : NCH₂CHN) ; 4,82 (mt, 1H : CHCOO) ; 6,36 (mt, 1H : ArNH) ; 6,68 (d large, J = 8 Hz, 1H : H 6) ; 6,92 (dd, J = 8 et 2 Hz, 1H : H 6 de l'indanyle) ; 7,02 (d, J = 2 Hz, 1H : H 4 de l'indanyle) ; 7,26 (d, J = 8 Hz, 1H : H 7 de l'indanyle) ; 7,32 (t, J = 8 Hz, 1H : H 7) ; de 7,45 à 7,65 (mt, 3H : H 2 - H 3 et H 8) ; 8,26 (d large, J = 8,5 Hz, 1H : H 4) ; 9,10 (d, J = 7,5 Hz, 1H : ArCONH).
- analyse élémentaire : C₂₈H₃₃N₃O₃S₂, CF₃CO₂H :

| | | | | |
|---|---|---|---|---|
| Calculé (%) | C = 56,5 ; | H = 5,37 ; | N = 6,59 ; | S = 10,06 |
| Trouvé (%) | C = 56,2 ; | H = 5,6 ; | N = 6,6 ; | S = 10,1 |

### EXEMPLE 8

A une solution de 0,3 g de la N-[5-(2(R)-tert-butoxycarbonylamino-3-triphénylméthylthio-propylamino)-naphtyl-1-carbonyl]-L-méthionine dans 5 cm3 de diéthyléther on ajoute 0,048 g d'alcool benzylique, puis 0,054 g de 4-diméthylaminopipéridine et 0,1 g de 1,3-dicyclohexylcarbodiimide. Le milieu réactionnel est agité à une température voisine de 20°C jusqu'à estérification complète, puis filtré sur un verre fritté et lavé successivement trois fois par 100 cm3 d'eau, puis par 100 cm3 d'une solution aqueuse d'acide acétique à 5 % (v/v), puis par 100 cm3 d'eau et enfin deux fois par 100 cm3 d'une solution aqueuse d'hydrogénocarbonate de sodium à 10 % (p/v). La phase organique est séchée sur du sulfate de magnésium, filtrée et concentrée à sec sous pression réduite. On obtient 0,21 g de l'ester benzylique de la N-[5-(2(R)-tert-butoxycarbonylamino-3-triphénylméthylthio-propylamino)-naphtyl-1-carbonyl]-L-méthionine qui sont utilisés dans l'étape suivante sans autre purification.

En opérant comme dans l'exemple 3 pour la préparation du trifluoroacétate de la N-[5-(2(R)-amino-3-mercapto-propylamino)-naphtyl-1-carbonyl]-L-méthionine mais à partir de 0,21 g de l'ester benzylique de la N-[5-(2(R)-tert-butoxycarbonylamino-3-triphénylméthylthio-propylamino)-naphtyl-1-carbonyl]-L-méthionine, on obtient 0,021 g du trifluoroacétate de l'ester benzylique de la N-[5-(2(R)-amino-3-mercapto-propylamino)-naphtyl-1-carbonyl]-L-méthionine sous forme d'une poudre dont les caractéristiques sont les suivantes :
- spectre de résonance magnétique nucléaire du proton (200 MHz, (CD₃)₂SO d6 avec ajout de quelques gouttes de CD₃COOD d4, δ en ppm) : 2,10 (mt, 2H : CH₂) ; 2,10 (s, 3H : SCH₃) ; de 2,50 à 3,00 (mt, 4H : CH₂S) ; de 3,10 à 3,90 (mt, 3H : NCH₂CHN) ; 4,73 (mt, 1H : CHCOO) ; 5,23 (AB limite, J = 13,5 Hz, 2H : COOCH₂Ar) ; 6,32 (mt résiduel : ArNH) ; 6,70 (d, J = 7,5 Hz, 1H : H 6) ; de 7,20 à 7,60 (mt, 9 H : H aromatiques - H 2 - H 3 - H 7 et H 8) ; 8,27 (d large, J = 7,5 Hz, 1H : H 4) ; 8,98 (d résiduel, J = 7,5 Hz : ArCONH).
- analyse élémentaire : C₂₆H₃₁N₃O₃S₂, 1,25 CF₃CO₂H :

| | | | | |
|---|---|---|---|---|
| Calculé (%) | C = 53,47 ; | H = 5,08 ; | N = 6,56 ; | S = 10,02 |
| Trouvé (%) | C = 53,3 ; | H = 4,9 ; | N = 6,7 ; | S = 10,2 |

### EXEMPLE 9

En opérant comme dans l'exemple 8 pour la préparation de l'ester benzylique de la N-[5-(2(R)-tert-butoxycarbonylamino-3-triphénylméthylthiopropylamino)-naphtyl-1-carbonyl]-L-méthionine mais à partir de 0,053 g de 2-(2-méthoxyéthoxy)éthanol, on obtient 0,43 g de produit qui purifiée par chromatographie sur silice [éluant: cyclohexane-acétate d'éthyle (70-30 en volumes) conduit à 0,31 g d'ester 2-(2-méthoxyéthoxy)éthylique de la N-[5-(2(R)-tert-butoxycarbonylamino-3-tiphénylméthylthio-propylamino)-naphtyl-1-carbonyl]-L-méthionine dont les caractéristiques sont les suivantes :
- spectre de résonance magnétique nucléaire du proton (200 MHz, CDCl₃, δ en ppm) : 1,45 (s, 9H : OC(CH₃)₃) ; 2,12 (mt, 2H : CH₂) ; 2,12 (s, 3H : SCH₃) ; 2,54 et 2,64 (2 mts, 2H chacun : CH₂S) ; de 3,05 à 3,30 (mt, 2H : NCH₂) ; 3,36 (s, 3H : COOCH₃) ; de 3,40 à 4,05 (mt, 7H : OCH₂ et CHN) ; 4,37 (mt, 2H : COOCH₂) ; 4,68 (d, J = 8 Hz, 1H : NHCOO) ; 5,05 (mt, 1H : CHCOO) ; 6,52 (d large, J = 7,5 Hz, 1H : H 6) ; 6,68 (d, J = 8 Hz, 1H : ArCONH) ; de 7,10 à 7,90 (mt, 21H : SC(C₆H₅)₃ - H 2 - H 3 - H 4 - H 7 - H 8 et ArNH).

En opérant comme dans l'exemple 3 pour la préparation du trifluoroacétate de la N-[5-(2(R)-amino-3-mercapto-propylamino)-naphtyl-1-carbonyl]-L-méthionine mais à partir de 0,21 g de l'ester 2-(2-méthoxyéthoxy)éthylique de la N-[5-(2(R)-tert-butoxycarbonylamino-3-triphénylméthylthiopropylamino)-naphtyl-1-carbonyl]-L-méthionine, on obtient 0,051 g du trifluoroacétate de l'ester 2-(2-méthoxyéthoxy) éthylique de la N-[5-(2(R)-amino-3-mercapto-propylamino)-naphtyl-1-carbonyl]-L-méthionine dont les caractéristiques sont les suivantes :
- spectre de résonance magnétique nucléaire du proton (200 MHz, (CD₃)₂SO d6, δ en ppm) : 2,10 (mt, 2H : CH₂) ; 2,12 (s, 3H : SCH₃) ; 2,64 (t, J = 7,5 Hz, 2H : CH₂S) ; 2,90 (AB limite, 2H : CH₂S) ; 3,25 (s, 3H : OCH₃); de 3,30 à 3,80 (mt, 9H : OCH₂ et NCH₂CHN) ; 4,28 (mt, 2H : COOCH₂) ; 4,67 (mt, 1H : CHCOO) ; 6,32 (mf, 1H : ArNH) ; 6,72 (d large, J = 8 Hz, 1H : H 6); 7,37 (t, J = 8 Hz, 1H : H 7) ; de 7,40 à 7,65 (mt, 3H : H 2 - H 3 et H 8) ; 8,09 (mf, 3H : NH₃⁺CF₃COO⁻) ; 8,28 (d large, J = 8 Hz, 1H : H 4) ; 8,87 (d, J = 7,5 Hz, 1H : ArCONH).
- analyse élémentaire : C₂₄H₃₅N₃O₅S₂, 1,25 CF₃CO₂H :

| | | | | |
|---|---|---|---|---|
| Calculé (%) | C = 48,8 ; | H = 5,60 ; | N = 6,44 ; | S = 9,83 |
| Trouvé (%) | C = 48,8 ; | H = 5,7 ; | N = 6,6 ; | S = 10,0 |

### EXEMPLE 10

En opérant comme dans l'exemple 7 pour la préparation de l'ester de 5-indanyle de la N-tert-butoxycarbonyl-L-méthionine mais à partir de 3,73 g de 2-(phénylsulfonyl)éthanol, on obtient 7,9 g d'ester 2-(phénylsulfonyl)éthylique de la N-tert-butoxycarbonyl-L-méthionine qui sont utilisés dans l'étape suivante sans autre purification.

En opérant comme dans l'exemple 7 pour la préparation de l'ester de 5-indanyle de la L-méthionine mais à partir de 4,17 g d'ester 2-(phénylsulfonyl)éthylique de la N-tert-butoxycarbonyl-L-méthionine, on obtient 3,54 g d'ester 2-(phénylsulfonyl)éthylique de la L-méthionine qui sont utilisés dans l'étape suivante sans autre purification.

En opérant comme dans l'exemple 1 pour la préparation de l'ester méthylique de la N-[(5-nitro-naphtyl)-1-carbonyl]-L-méthionine, mais à partir de 2,75 g d'ester 2-(phénylsulfonyl)éthylique de la L-méthionine, on obtient 2,06 g d'ester 2-(phénylsulfonyl)éthylique de la N-[(5-nitro-naphtyl)-1-carbonyl]-L-méthionine dont les caractéristiques sont les suivantes :
- spectre de résonance magnétique nucléaire du proton (300 MHz, (CD₃)₂SO d6, δ en ppm) : 1,92 (mt, 2H : CH₂) ; 2,06 (s, 3H : SCH₃) ; 2,55 (mt, 2H : CH₂S) ; 3,81 (t, J = 6 Hz, 2H : CH₂SO₂) ; de 4,35 à 4,55 (mt, 2H : COOCH₂) ; 4,48 (mt, 1H : CHCOO) ; 7,68 (t, J = 7,5 Hz, 2H : H aromatiques en méta du sulfonyle) ; de 7,75 à 7,95 (mt, 4H : H 2 - H 3 - H 7 et H aromatique en para du sulfonyle) ; 7,96 (d, J= 7,5 Hz, 2H : H aromatiques en ortho du sulfonyle) ; 8,34 (dd, J = 7,5 et 1,5 Hz, 1H : H 4) ; 8,40 et 8,53 (2 d larges, J = 8 Hz, 1H chacun : H 6 et H 8) ; 9,09 (d, J = 7,5 Hz, 1H : ArCONH).

En opérant comme dans l'exemple 1 pour la préparation de l'ester méthylique de la N-[(5-amino-naphtyl)-1-carbonyl]-L-méthionine, mais à partir 2,06 g de l'ester 2-(phénylsulfonyl)éthylique de la N-[(5-nitro-naphtyl)-1-carbonyl]-L-méthionine, on obtient 3,40 g de l'ester 2-(phénylsulfonyl)éthylique de la N-[(5-amino-naphtyl)-1-carbonyl]-L-méthionine qui sont utilisés dans l'étape suivante sans autre purification.

En opérant comme dans l'exemple 3 pour la préparation de l'ester méthylique de la N-[5-(2(R)-tert-butoxycarbonylamino-3-triphénylméthylthiopropylamino)-naphtyl-1-carbonyl]-L-méthionine mais à partir 0,972 g de l'ester 2-(phénylsulfonyl)éthylique de la N-[(5-amino-naphtyl)-1-carbonyl]-L-méthionine, on obtient 0,21 g de l'ester 2-(phénylsulfonyl)éthylique de la N-[5-(2(R)-tert-butoxycarbonylamino-3-triphénylméthylthio-propylamino)-naphtyl-1-carbonyl]-L-méthionine qui sont utilisés dans l'étape suivante sans autre purification.

En opérant comme dans l'exemple 3 pour la préparation du trifluoroacétate de la N-[5-(2(R)-amino-3-mercapto-propylamino)-naphty]-1-carbonyl]-L-méthionine mais à partir de 0,21 g de l'ester 2-(phénylsulfonyl)éthylique de la N-[5-(2(R)-tert-butoxycarbonylamino-3-triphénylméthylthio-propylamino)-naphtyl-1-carbonyl]-L-méthionine, on obtient 0,013 g du trifluoroacétate de l'ester 2-(phénylsulfonyl)éthylique de la N-[5-(2(R)-amino-3-mercapto-propylamino)-naphty]-1-carbonyl]-L-méthionine dont les caractéristiques sont les suivantes :
- spectre de résonance magnétique nucléaire du proton (400 MHz, (CD₃)₂SO d6 avec ajout de quelques gouttes de CD₃COOD d4, δ en ppm) : 1,90 (mt, 2H : CH₂) ; 2,05 (s, 3H : SCH₃) ; 2,54 (mt : CH₂S) ; 2,88 (mt, 2H : CH₂S) ; de 3,40 à 3,65 (mt, 3H : NCH₂CHN) ; 3,76 (AB limite, 2H : CH₂SO₂) ; 4,38 et 4,45 (AB limite, 1H chacun : COOCH₂) ; 4,46 (mt, 1H : CHCOO) ; 6,70 (d, J = 7,5 Hz, 1H : H 6) ; 7,36 (t, J = 7,5 Hz, 1H : H 7) ; de 7,45 à 7,60 (mt, 3H : H 2 - H 3 et H 8) ; 7,68 (t, J = 7,5 Hz, 2H : H aromatiques en méta du sulfonyle) ; 7,78 (t, J = 7, 5 Hz, 1H : H aromatique en para du sulfonyle) ; 7,96 (d, J= 7,5 Hz, 2H : H aromatiques en ortho du sulfonyle) ; 8,26 (d large, J = 8 Hz, 1H : H 4) ; 8,76 (d résiduel, J = 7,5 Hz : ArCONH).
- spectre de masse (DIC) : M/Z = 576 (MH⁺).

### EXEMPLE 11

A une suspension de 4,7 g d'acide 5-nitro-1-naphtoïque dans 50 cm3 de méthanol, on ajoute 1 cm3 d'acide sulfurique à 97 %, puis on porte au reflux pendant 12 heures. La solution est refroidie à une température voisine de 20°C, le précipité formé est essoré et lavé deux fois par 5 cm3 de méthanol glacé, puis séché jusqu'à poids constant. On obtient 4,56 g de 5-nitro-1-naphtoate de méthyle fondant à 105°C.

A une solution de 4,45 g de 5-nitro-1-naphtoate de méthyle dans un mélange de 150 cm3 d'acétate d'éthyle et 35 cm3 d'éthanol on ajoute 21,5 g de dihydrate de chlorure d'étain (II). La solution est chauffée à une température voisine de 70°C pendant 30 minutes, puis refroidie à une température voisine de 20°C. Le mélange réactionnel est versé sur 200 cm3 de glace, le pH de la solution est progressivement amené à 7-8 par addition d'une solution d'hydrogénocarbonate de sodium à 5 % (p/v). Le mélange obtenu est filtré sur un verre fritté garni de célite, la phase organique est séparée par décantation et la phase aqueuse est extraite 2 fois par 150 cm3 d'acétate d'éthyle. Les phases organiques sont réunies, séchées sur du sulfate de magnésium, filtrées et concentrées à sec sous pression réduite. On obtient 4,5 g de 5-amino-1-naphtoate de méthyle sous forme d'une huile qui sont utilisés dans l'étape suivante sans autre purification.

A une solution de 4,5 g de 5-amino-1-naphtoate de méthyle dans 30 cm3 de méthanol on ajoute 14,75 g de S-triphénylméthyl-N-tert-butoxycarbonyl-cystéinal, 0,14 cm3 d'acide acétique concentré, du tamis moléculaires (3Å) puis 4,15 g de cyanoborohydrure de sodium. Le mélange réactionnel est agité pendant 2 jours à une température voisine de 20°C puis filtré sur verre fritté garni de célite. Le verre fritté est lavé par du méthanol. Le filtrat est concentré sous pression réduite, redissous dans 150 cm3 d'acétate d'éthyle et lavé par 100 cm3 d'une solution aqueuse d'hydrogénocarbonate de sodium à 10 % (p/v), par 100 cm3 d'une solution aqueuse d'acide citrique à 10 % (p/v), par 100 cm3 d'eau distillée, par 100 cm3 d'une solution aqueuse d'hydrogénocarbonate de sodium 10 % (p/v), et enfin par 100 cm3 d'une solution aqueuse saturée de chlorure de sodium. La phase organique est séchée sur du sulfate de magnésium, filtrée et concentrée à sec sous pression réduite. Le produit est purifié par chromatographie sur silice [éluant : cyclohexane-acétate d'éthyle (85-15 en volumes)]. On obtient 3,5 g de 5-(2(R)-tert-butoxycarbonylamino-3-triphénylméthylthio-propylamino)-1-naphtoate de méthyle sous forme d'une meringue beige qui sont utilisés dans l'étape suivante sans autre purification.

A une solution de 1,5 g de 5-(2(R)-tert-butoxycarbonylamino-3-triphénylméthylthio-propylamino)-1-naphtoate de méthyle dans 25 cm3 de diméthylformamide, on ajoute sous agitation 3,6 cm3 d'iodure de méthyle et 7 g d'hydrogénocarbonate de sodium solide. Le mélange réactionnel est agité pendant 20 heures à une température voisine de 20°C puis versé sur 200 cm3 de glace. La phase aqueuse est extraite 3 fois par 150 cm3 d'acétate d'éthyle. Les phases organiques sont réunies, séchées sur du sulfate de magnésium, filtrées et concentrées à sec sous pression réduite. L'huile rouge obtenue est purifiée par chromatographie sur silice [éluant : cyclohexane-acétate d'éthyle (90-10 en volumes)]. On obtient 0,56 g de 5-(2(R)-tert-butoxycarbonylamino-3-triphénylméthylthio-propyl-N-méthyl-amino)-1-naphtoate de méthyle dont les caractéristiques sont les suivantes :
- spectre de résonance magnétique nucléaire du proton (400 MHz, (CD₃)₂SO d6, δ en ppm) : 1,42 (s, 9H : OC(CH₃)₃) ; 2,28 (mt, 2H : SCH₂) ; 2,62 (s, 3H : NCH₃) ; 2,85 et 3,04 (2 dd, respectivement J = 13 et 7 Hz et J = 13 et 6.5 Hz, 1H chacun : NCH₂) ; 3,82 (mt, 1H : CHN) ; 3,96 (s, 3H : COOCH₃) ; 6,83 (d, J = 9 Hz, 1H : NHCOO) ; 7,15 (d, J = 8 Hz, 1H : H 6) ; de 7,20 à 7,40 (mt, 15H : SC(C₆H₅)₃) ; 7,47 et 7,52 (2 t, J = 8 Hz, 1H : H 3 et H 7) ; de 8,10 à 8,15 et 8,36 (respectivement mt et d, J = 8 Hz, 2H et 1H : H 2 - H 4 et H 8).

A une solution de 0,56 g de 5-(2(R)-tert-butoxycarbonylamino-3-triphénylméthylthio-propyl-N-méthyl-amino)-1-naphtoate de méthyle dans 25 cm3 d'eau distillée et 55 cm3 d'éthanol, on ajoute 0,2 g de potasse. La solution est chauffée au reflux pendant 2 heures, puis concentrée sous pression réduite. Le résidu est redissous dans de l'eau distillée puis amené à pH 3 avec une solution aqueuse d'acide citrique à 10 % (p/v). La phase aqueuse est extraite 3 fois par 50 cm3 d'acétate d'éthyle. Les phases organiques sont réunies, lavées avec une solution aqueuse saturée de chlorure de sodium, séchées sur du sulfate de magnésium, filtrées et concentrées à sec sous pression réduite. On obtient 0,55 g d'acide 5-(2(R)-tert-butoxycarbonylamino-3-triphénylméthylthio-propyl-N-méthyl-amino)-1-naphtoïque, sous forme d'une huile jaune, qui sont utilisés dans l'étape suivante sans autre purification.

En opérant comme dans l'exemple 1 pour la préparation de l'ester méthylique de la N-[(5-nitro-naphtyl)-1 carbonyl]-L-méthionine mais à partir de 0,55 g d'acide 5-(2(R)-tert-butoxycarbonylamino-3-triphénylméthylthio-propyl-N-méthyl-amino)-1-naphtoïque, on obtient 1,2 g d'une huile orange qui, purifiée par chromatographie sur silice [éluant : cyclohexane-acétate d'éthyle (80-20 en volumes)], conduit à 0,55 g de l'ester méthylique de la N-[5-(2(R)-tert-butoxycarbonylamino-3-triphénylméthylthio-propyl-N-méthyl-amino)-naphtyl-1-carbonyl]-L-méthionine dont les caractéristiques sont les suivantes :
- spectre de résonance magnétique nucléaire du proton (300 MHz, (CD₃)₂SO, δ en ppm) : 1,41 (s, 9H : OC(CH₃)₃) ; 2,06 (mt, 2H : CH₂) ; 2,10 (s, 3H : SCH₃) ; 2,38 (mt, 2H : SCH₂) ; 2,60 (mt, 2H : CH₂S) ; 2,60 (s, 3H : NCH₃) ; 2,85 et 3,04 (2 mts, 1H chacun: NCH₂) ; 3,75 (s, 3H : COOCH₃) ; 3,82 (mt, 1H : CHN) ; 4,69 (mt, 1H : CHCOO) ; 6,85 (d, J = 9 Hz, 1H : NHCOO) ; 7,10 (d, J = 8 Hz, 1H : H 6) ; de 7,20 à 7,45 et 7,42 (2 mts, respectivement 16H et 1H : SC(C₆H₅)₃ - H 3 et H 7) ; 7,55 (d, J = 8 Hz, 1H : H 2); 7,85 et 7,92 (2 d, J = 8 Hz, 1H chacun : H 4 et H 8) ; 8,91 (d, J = 7.5 Hz, 1H : ArCONH).

En opérant comme dans l'exemple 3 pour la préparation de la N-[5-(2(R)-tert-butoxycarbonylamino-3-triphénylméthylthio-propylamino)-naphtyl-1-carbonyl]-L-méthionine mais à partir de 0,3 g de l'ester méthylique de la N-[5-(2(R)-tert-butoxycarbonylamino-3-triphénylméthylthio-propyl-N-méthyl-amino)-naphtyl-1-carbonyl]-L-méthionine. On obtient 0,3 g de la N-[5-(2(R)-tert-butoxycarbonylamino-3-triphénylméthylthio-propyl-N-méthyl-amino)-naphtyl-1-carbonyl]-L-méthionine qui sont utilisés dans l'étape suivante sans autre purification.

En opérant comme dans l'exemple 3 pour la préparation du trifluoroacétate de la N-[5-(2(R)-amino-3-mercapto-propylamino)-naphtyl-1-carbonyl]-L-méthionine mais à partir de 0,25g de la N-[5-(2(R)-tert-butoxycarbonylamino-3-triphénylméthylthio-propyl-N-méthyl-amino)-naphtyl-1-carbonyl]-L-méthionine, on obtient 0,045 g du trifluoroacétate de la N-[5-(2(R)-amino-3-mercapto-propyl-N-méthyl-amino)-naphtyl-1-carbonyl]-L-méthionine sous forme d'une poudre dont les caractéristiques sont les suivantes :
- spectre de résonance magnétique nucléaire du proton (400 MHz, (CD₃)₂SO d6, δ en ppm) : de 1,95 à 2,15 (mt, 2H : CH₂) ; 2,10 (s, 3H : SCH₃) ; 2,63 (mt, 2H : SCH₂) ; 2,78 (s, 3H : NCH₃) ; 2,84 (mt, 2H : CH₂S) ; 3,28 et 3,40 (2 dd, respectivement J = 11 et 8 Hz et J = 11 et 6 Hz, 1H chacun : NCH₂) ; 3,57 (mt, 1H : CHN) ; 4,60 (mt, 1H : CHCOO) ; 7,35 (d, J = 7,5 Hz, 1H : H 6) ; 7,53 (t, J = 8 Hz; 1H : H 7) ; de 7,55 à 7,70 (mt, 2H : H 2 et H 3) ; 7,95 (mf, 2 H : NH₂) ; 7,99 (d, J = 8 Hz, 1H : H 8) ; 8,55 (dd, J = 8 et 2 Hz, 1H : H 4) ; 8,83 (d, J = 7.5 Hz, 1H : ArCONH).
- analyse élémentaire : C₂₀H₂₇N₃O₃S₂, 1,25 CF₃CO₂H :

| | | | | |
|---|---|---|---|---|
| Calculé (%) | C = 47,9 ; | H = 5,05 ; | N = 7,45 ; | S = 11,37 |
| Trouvé (%) | C = 47,7 ; | H = 5,3 ; | N = 7,4 | S = 11,4 |

### EXEMPLE 12

En opérant comme dans l'exemple 3 pour la préparation du trifluoroacétate de la N-[5-(2(R)-amino-3-mercapto-propylamino)-naphtyl-1-carbonyl]-L-méthionine mais à partir de 0,25 g de l'ester méthylique de la N-[5-(2(R)-tert-butoxycarbonylamino-3-triphénylméthylthiopropyl-N-méthyl-amino)-naphtyl-1-carbonyl]-L-méthionine, on obtient 0,049 g du trifluoroacétate de l'ester méthylique de la N-[5-(2(R)-amino-3-mercapto-propyl-N-méthyl-amino)-naphtyl-1-carbonyl]-L-méthionine sous forme d'une poudre dont les caractéristiques sont les suivantes :
- spectre de résonance magnétique nucléaire du proton (250 MHz, (CD₃)₂SO d6 avec ajout de quelques gouttes de CD₃COOD d4, δ en ppm) : 2,06 (mt, 2H : CH₂) ; 2,10 (s, 3H : SCH₃) ; 2,63 (mt, 2H : SCH₂) ; 2,80 (s, 3H : NCH₃) ; 2,84 (mt, 2H : CH₂S) ; 3,30 et 3,42 (2 dd, J = 13,5 et 9 Hz et J = 13,5 et 5,5 Hz, 1H chacun : NCH₂) ; 3,59 (mt, 1H : CHN) ; 3,75 (s, 3H : COOCH₃) ; 4,70 (mt, 1H : CHCOO) ; 7,35 (d, J = 7,5 Hz, 1H : H 6) ; de 7,50 à 7,70 (mt, 3H : H 2 - H 3 et H 7) ; 7,97 (d, J = 8,5 Hz, 1H : H 8) ; 8,53 (dd, J = 7,5 et 2 Hz, 1H : H 4) ; 8,93 (d, J = 7.5 Hz, 1H : ArCONH).
- analyse élémentaire : C₂₁H₂₉N₃O₃S₂, 1,25 CF₃CO₂H :

| | | | | |
|---|---|---|---|---|
| Calculé (%) | C = 48,82 ; | H = 5,27 ; | N = 7,27 ; | S = 11,09 |
| Trouvé (%) | C = 48,3 ; | H = 5,1 ; | N = 7,0 ; | S = 11,2 |

### EXEMPLE 13

A une suspension de 3,75 g de S-triphénylméthyl-L-cystéine dans 15 cm3 d'eau, on ajoute sous agitation 1,5 cm3 de soude à 33 % (p/p) suivie de 1,5 cm3 de diméthylsulfate. Le mélange réactionnel est chauffé sous agitation à 90°C jusqu'à obtention d'une solution limpide, puis chauffé au reflux pendant 2 heures. Après refroidissement le précipité obtenu est filtré, lavé à l'eau puis au diéthyléther On obtient ainsi 1,95 g de N-méthyl-S-triphénylméthyl-L-cystéine fondant à 114°C.

A une solution de 1,95 g de N-méthyl-S-triphénylméthyl-L-cystéine dans 20 cm3 de dichlorométhane on ajoute 0.68 cm3 de triéthylamine et 1,06 g de di-tert-butyl dicarbonate. Le mélange réactionnel est agité pendant 20 heures à une température voisine de 20°C puis lavé par une solution aqueuse d'acide citrique à 10 % (p/v) et enfin par de l'eau distillée. La phase organique est séchée sur sulfate de magnésium, filtrée et concentrée sous pression réduite. Le solide beige obtenu est purifié par chromatographie sur silice [éluant : acétate d'éthyle]. On obtient 0,4 g de N-méthyl-N-tert-butoxycarbonyl-S-triphénylméthyl-L-cystéine dont les caractéristiques sont les suivantes :
- spectre de résonance magnétique nucléaire du proton : (250 MHz, (CD₃)₂SO d6 avec ajout de quelques gouttes de CD₃COOD d 4, à une température de 393 K, δ en ppm) : 1,40 (s, 9H : OC(CH₃)₃) ; de 2,55 à 2,82 (mt, 2H : SCH₂) ; 2,68 (s, 3H : NCH₃) ; 4,16 (dd, J = 10 et 5,5 Hz, 1H : CHN) ; de 7,20 à 7,45 (mt, 15H : H aromatiques).

A une suspension de 1,57 g de N,O diméthylhydroxylamine dans 10 cm3 de dichlorométhane à une température voisine de 0°C, on ajoute 1,96 cm3 de N-méthyl pipéridine. Cette solution est additionnée au mélange réactionnel contenant 7,8 g de N-méthyl-N-tert-butoxycarbonyl-S-triphénylméthyl-L-cystéine, 1,96 cm3 de N-méthyl pipéridine et 2,08 cm3 d'isobutylchloroformiate dans 20 cm3 de dichlorométhane à une température voisine de -8°C. Le mélange réactionnel est agité pendant 2 heures à une température voisine de 20°C, puis lavé par une solution aqueuse d'acide citrique à 10 % (p/v), puis par une solution aqueuse d'hydroxyde de sodium 2 % (p/v) et enfin par une solution saturée de chlorure de sodium. La phase organique est séchée sur sulfate de magnésium, filtrée et concentrée sous pression réduite. L'huile épaisse rouge obtenue est purifiée par chromatographie sur silice [éluant : dichlorométhane-acétate d'éthyle (95-5 en volumes)]. On obtient 4,2 g de N-(N-méthyl-2(R)-tert-butoxycarbonylamino-3-triphénylméthylthio-propionyl)-N,O-diméthyl-hydroxylamine sous forme d'une huile rouge dont les caractéristiques sont les suivantes :
- spectre de résonance magnétique nucléaire du proton : (200 MHz, (CD₃)₂SO d6 avec ajout de quelques gouttes de CD₃COOD d 4, à une température de 373 K, δ en ppm) : 1,41 (s, 9H : OC(CH₃)₃) ; de 2,35 à 2,75 (mt, 2H : SCH₂) ; 2,62 (s, 3H : NCH₃) ; 3,06 (s, 3H : NCH₃) ; 3,60 (s, 3H : NOCH₃) ;4,89 (dd, J = 9,5 et 6 Hz, 1H : CHN) ; de 7,20 à 7,45 (mt, 15H : H aromatiques).

A une suspension de 0,386 g d'hydrure de lithium et d'aluminium dans 35 cm3 de diéthyléther à une température voisine de -45°C, on ajoute 4,2 g de N-(-N-méthyl-2 (R)-tert-butoxycarbonylamino-3-triphénylméthylthio-propionyl)-N,O-diméthyl-hydroxylamine dans 15 cm3 de diéthyléther. On laisse, sous agitation, la température remonter vers 0°C. Après refroidissement à une température voisine de -35°C, on ajoute une solution de 1,77 g d'hydrogénocarbonate de sodium dans 6,5 cm3 d'eau. La phase organique est lavée 3 fois par 10 cm3 d'une solution d'acide chlorhydrique normale, puis 2 fois par 10 cm3 d'une solution saturée d'hydrogénocarbonate de sodium et enfin par 10 cm3 d'une solution saturée de chlorure de sodium. La phase organique est séchée sur sulfate de magnésium, filtrée et concentrée sous pression réduite. On obtient 2,2 g de N-méthyl-N-tert-butoxycarbonyl-S-triphénylméthyl-cystéinal qui sont utilisés dans l'étape suivante sans autre purification.

En opérant comme dans l'exemple 3 pour la préparation de l'ester méthylique de la N-[5-(2(R)-tert-butoxycarbonylamino-3-triphénylméthylthiopropylamino)-naphtyl-1-carbonyl]-L-méthionine mais à partir de 2,9 g de N-méthyl-N-tert-butoxycarbonyl-S-triphénylméthyl-cystéinal, on obtient 0,55 g de l'ester méthylique de la N-{5-[(N-méthyl-2(R)-tert-butoxycarbonylamino)-3-triphénylméthylthiopropylamino]-naphtyl-1-carbonyl}-L-méthionine qui sont utilisés dans l'étape suivante sans autre purification.

En opérant comme dans l'exemple 3 pour la préparation de la N-[5-(2(R)-tert-butoxycarbonylamino-3-triphénylméthylthio-propylamino)-naphtyl-1-carbonyl]-L-méthionine, mais à partir de 0,35 g d'ester méthylique de la N-{5-[(N-méthyl-2(R)-tert-butoxycarbonylamino)-3-triphénylméthylthio-propylamino]-naphtyl-1-carbonyl}-L-méthionine, on obtient 0,33 g de N-(5-[(N-méthyl-2(R)-tert-butoxycarbonylamino)-3-triphénylméthylthio-propylamino]-naphtyl-1-carbonyl}-L-méthionine qui sont utilisés dans l'étape suivante sans autre purification.

En opérant comme dans l'exemple 3 pour la préparation du trifluoroacétate de la N-[5-(2(R)-amino-3-mercapto-propylamino)-naphtyl-1-carbonyl]-L-méthionine, mais à partir de 0,33 g de la N-(5-[(N-méthyl-2(R)-tert-butoxycarbonylamino)-3-triphénylméthylthio-propylamino]-naphtyl-1-carbonyl}-L-méthionine, on obtient 0,05 g de trifluoroacétate de la N-[5-(2(R)-méthylamino-3-mercapto-propylamino)-naphtyl-1-carbonyl]-L-méthionine dont les caractéristiques sont les suivantes :
- spectre de résonance magnétique nucléaire du proton (300 MHz, (CD₃)₂SO d6 avec ajout de quelques gouttes de CD₃COOD d4, δ en ppm) : 2,08 (mt, 2H : CH₂) ; 2,08 (s, 3H : SCH₃) ; 2,64 (mt, 2H : CH₂S) ; 2,68 (s, 3H : NCH₃) ; 2,92 (mt, 2H : CH₂S) ; de 3,30 à 3,65 (mt, 3H : NCH₂CHN) ; 4,58 (mt, 1H : CHCOO) ; 6,73 (d, J = 8 Hz, 1H : H 6) ; 7,35 (t, J = 8 Hz, 1H : H 7) ; de 7,45 à 7,65 (mt, 3H : H 2 - H 3 et H 8) ; 8,24 (d large, J = 8,5 Hz, 1H : H 4) ; 8,71 (d résiduel, J = 7,5 Hz : ArCONH).
- analyse élémentaire : C₂₀H₂₇N₃O₃S₂, 1,25 CF₃CO₂H :

| | | | | |
|---|---|---|---|---|
| Calculé (%) | C = 47,91 ; | H = 5,05 ; | N = 7,45 ; | S = 11,37 |
| Trouvé (%) | C = 47,8 ; | H = 4,7 ; | N = 7,5 ; | S = 11,5 |

### EXEMPLE 14

En opérant comme dans l'exemple 3 pour la préparation du trifluoroacétate de la N-[5-(2(R)-amino-3-mercapto-propylamino)-naphtyl-1-carbonyl]-L-méthionine mais à partir de 0,35 g de l'ester méthylique de la N-{5-[(N-méthyl-2(R)-tert-butoxycarbonylamino)-3-triphénylméthylthio-propylamino]-naphtyl-1-carbonyl}-L-méthionine, on obtient 0,049 g du trifluoroacétate de l'ester méthylique de la N-[5-(2(R)-méthylamino-3-mercapto-propylamino)-naphtyl-1-carbonyl]-L-méthionine sous forme d'une poudre dont les caractéristiques sont les suivantes :
- spectre de résonance magnétique nucléaire du proton (400 MHz, (CD₃)₂SO d6 avec ajout de quelques gouttes de CD₃COOD d4, δ en ppm) : 2,25 et 2,40 (2 mts, 1H chacun : CH₂) ; 2,69 (s, 3H : SCH₃) ; 2,94 (mt, 2H : CH₂S) ; 2,94 (s, 3H : NCH₃) ; de 3,30 à 3,65 (mt, 5H : CH₂S et NCH₂CHN) ; 3,75 (s, 3H : COOCH₃) ; 4,72 (dd, J = 9 et 4,5 Hz, 1H : CHCOO) ; 6,73 (d, J = 8 Hz, 1H : H 6) ; 7,39 (t, J = 8 Hz, 1H : H 7) ; de 7,45 à 7,65 (mt, 3H : H 2 - H 3 et H 8) ; 8,28 (d large, J = 8 Hz, 1H : H 4).
- analyse élémentaire : C₂₁H₂₉N₃O₃S₂, 2,5 CF₃CO₂H :

| | | | | |
|---|---|---|---|---|
| Calculé (%) | C = 43,33 ; | H = 4,41 ; | N = 5,83 ; | S = 8,9 |
| Trouvé (%) | C = 43,9 ; | H = 4,6 ; | N = 5,9 ; | S = 8,7 |

### EXEMPLE 15

A une suspension de 12,5 g d'hydrure de potassium dans 80 cm3 de tétrahydrofuranne à une température voisine de 0°C, on ajoute 2,6 g d'éther couronne 18-6, puis au goutte à goutte une solution de 5 g d'ester méthylique de la N-tert-butoxycarbonyl-L-méthionine dans 15 cm3 de tétrahydrofuranne et enfin 2,5 cm3 d'iodure de méthyle. L'agitation est poursuivie à une température voisine de 0°C pendant 2 heures. On ajoute ensuite 6 cm3 d'acide acétique, puis le mélange réactionnel est coulé sur 200 g de glace. Le pH est amené à une valeur voisine de 9 par addition d'une solution d'hydroxyde de sodium à 10 % (p/v). Le mélange réactionnel est lavé trois fois par 20 cm3 de diéthyléther. La solution aqueuse est amené à un pH voisin de 3 par addition d'une solution d'acide chlorhydrique à 10 % (v/v), puis extraite trois fois par 20 cm3 d'acétate d'éthyle. Les phases organiques sont regroupées et lavées par une solution de chlorure de sodium saturée, séchées sur du sulfate de magnésium, filtrées et concentrées à sec sous pression réduite. On obtient 6 g d'ester méthylique de la N-méthyl-N-tert-butoxycarbonyl-L-méthionine qui sont utilisés dans l'étape suivante sans autre purification.

A une solution de 4,6 g d'ester méthylique de la N-méthyl-N-tert-butoxycarbonyl-L-méthionine dans 150 cm3 de méthanol, on ajoute 6,6 g de monohydrate d'acide para-toluènesulfonique et on porte au reflux pendant 24 heures en présence de tamis moléculaire (3Å). Le milieu réactionnel est refroidi à une température voisine de 20°C, puis filtré sur un verre fritté et le solvant est éliminé sous pression réduite. Le résidu est dissous dans 150 cm3 d'eau puis lavé par un mélange hexane-diéthyléther (50-50 en volumes). Le pH de la phase aqueuse est ensuite amené à une valeur voisine de 10 par addition d'une solution saturée d'hydrogénocarbonate de sodium. La solution aqueuse est extraite quatre fois par 50 cm3 d'acétate d'éthyle, les phases organiques sont regroupées et lavées par une solution de chlorure de sodium saturée, séchées sur du sulfate de magnésium, filtrées et concentrées à sec sous pression réduite. On obtient 6 g d'ester méthylique de la N-méthyl-L-méthionine dont les caractéristiques sont les suivantes :
- spectre de résonance magnétique nucléaire du proton (200 MHz, (CD₃)₂SO d6, d en ppm) : 1,75 (mt, 2H : CH₂) ; 1,95 (mf, 1H : NH) ; 2,04 (s, 3H : SCH₃) ; 2,22 (s, 3H : NCH₃) ; 2,52 (mt, 2H : CH₂S) ; 3,21 (dd, J = 7 et 5 Hz, 1H : NCHCOO) ; 3,66 (s, 3H : COOCH₃).

En opérant comme dans l'exemple 1 pour la préparation l'ester méthylique de la N-[(5-nitro-naphtyl)-1-carbonyl]-L-méthionine mais à partir de 0,68 g d'ester méthylique de la N-méthyl-L-méthionine, on obtient 1,34 g d'ester méthylique de la N-méthyl-N-[(5-nitro-naphtyl)-1-carbonyl]-L-méthionine qui sont utilisés dans l'étape suivante sans autre purification.

En opérant comme dans l'exemple 1 pour la préparation de l'ester méthylique de la N-[(5-amino-naphtyl)-1-carbonyl]-L-méthionine mais à partir de 1,17 g d'ester méthylique de la N-méthyl-N-[(5-nitro-naphtyl)-1-carbonyl]-L-méthionine, on obtient 0,97 g d'ester méthylique de la N-méthyl-N-[(5-aminonaphtyl)-1-carbonyl]-L-méthionine qui sont utilisés dans l'étape suivante sans autre purification.

En opérant comme dans l'exemple 3 pour la préparation de l'ester méthylique de la N-[5-(2(R)-tert-butoxycarbonylamino-3-triphénylméthylthiopropylamino)-naphtyl-1-carbonyl]-L-méthionine mais à partir de 0,95 g d'ester méthylique de la N-méthyl-N-[(5-amino-naphtyl)-1-carbonyl]-L-méthionine, on obtient 0,97 g d'ester méthylique de la N-méthyl-N-[5-(2(R)-tert-butoxycarbonylamino-3-triphénylméthylthio-propylamino)-naphtyl-1-carbonyl]-L-méthionine qui sont utilisés dans l'étape suivante sans autre purification.

En opérant comme dans l'exemple 3 pour la préparation de la N-[5-(2(R)-tert-butoxycarbonylamino-3-triphénylméthylthio-propylamino)-naphtyl-1-carbonyl]-L-méthionine, mais à partir de 0,37 g d'ester méthylique de la N-méthyl-N-[5-(2(R)-tert-butoxycarbonylamino-3-triphénylméthylthio-propylamino)-naphtyl-1-carbonyl]-L-méthionine on obtient 0,36 g de la N-méthyl-N-[5-(2(R)-tert-butoxycarbonylamino-3-triphénylméthylthio-propylamino)-naphtyl-1-carbonyl]-L-méthionine qui sont utilisés dans l'étape suivante sans autre purification.

En opérant comme dans l'exemple 3 pour la préparation de la N-[5-(2(R)-amino-3-mercapto-propylamino)-naphtyl-1-carbonyl]-L-méthionine mais à partir de 0.35 g de la N-méthyl-N-[5-(2(R)-tert-butoxycarbonylamino-3-triphénylméthylthiopropylamino)-naphtyl-1-carbonyl]-L-méthionine, on obtient 0,13 g de la N-méthyl-N-[5-(2(R)-amino-3-mercapto-propylamino)-naphtyl-1-carbonyl]-L-méthionine dont les caractéristiques sont les suivantes :
- spectre de résonance magnétique nucléaire du proton (400 MHz, (CD₃)₂SO d6 avec ajout de quelques gouttes de CD₃COOD d4, à une température de 413 K, δ en ppm) : de 1,90 à 2,40 (mt, 5H : CH₂ et SCH₃) ; de 2,40 à 2,90 (mt, 5H : CH₂S et NCH₃) ; 3,13 et 3,22 (2 dd, J = 14 et 6,5 Hz, 1H chacun : CH₂S) ; 3,56 et 3,76 (2 mts, respectivement 2H et 1H : NCH₂CHN) ; 5,10 (mf étalé, 1H : CHCOO) ; 6,68 (d, J = 8 Hz, 1H : H 6) ; de 7,10 à 7,50 (mt, 4H : H 2 - H 3 - H 7 et H 8) ; 8,15 (d large, J = 8 Hz, 1H : H 4).
- analyse élémentaire : C₂₀H₂₇N₃O₃S₂, 1,14 CF₃CO₂H :

| | | | | |
|---|---|---|---|---|
| Calculé (%) | C = 48,5 ; | H = 5,14 ; | N = 7,61 ; | S = 11,62 |
| Trouvé (%) | C = 48,4 ; | H = 5,2 ; | N = 7,6 ; | S = 11,6 |

### EXEMPLE 16

En opérant comme dans l'exemple 3 pour la préparation du trifluoroacétate de la N-[5-(2(R)-amino-3-mercapto-propylamino)-naphtyl-1-carbonyl]-L-méthionine mais à partir de 0,34 g d'ester méthylique de la N-méthyl-N-[5-(2(R)-tert-butoxycarbonylamino-3-triphénylméthylthio-propylamino)-naphtyl-1-carbonyl]-L-méthionine, on obtient 0,045 g de l'ester méthylique de la N-méthyl-N-[5-(2(R)-amino-3-mercapto-propylamino)-naphtyl-1-carbonyl]-L-méthionine sous forme d'une poudre dont les caractéristiques sont les suivantes :
- spectre de résonance magnétique nucléaire du proton (400 MHz, (CD₃)₂SO d6 avec ajout de quelques gouttes de CD₃COOD d4, à une température de 413°K, δ en ppm) : de 1,90 à 2,30 (mt, 5H : CH₂ et SCH₃) ; de 2,40 à 2,90 (mt, 5H : CH₂S et NCH₃) ; 3,13 et 3,22 (2 dd, J = 14 et 6,5 Hz, 1H chacun : CH₂S) ; de 3,45 à 3,80 (mt, 3H : NCH₂CHN) ; 3,70 (s, 3H : COOCH₃) ; 5,10 (mf étalé, 1H : CHCOO) ; 6,68 (d, J = 8 Hz, 1H : H 6) ; de 7,10 à 7,50 (mt, 4H : H 2 - H 3 - H 7 et H 8) ; 8,15 (d large, J = 8 Hz, 1H : H 4).
- analyse élémentaire : C₂₁H₂₉N₃O₃S₂, 1,14 CF₃CO₂H :

| | | | | |
|---|---|---|---|---|
| Calculé (%) | C = 49,42 ; | H = 5,37 ; | N = 7,43 ; | S = 11,33 |
| Trouvé (%) | C = 49,4 | H = 5,0 ; | N = 7,5 ; | S = 11,5 |

### EXEMPLE 17

On prépare l'acide 6-nitro-naphtalène-1-carboxylique selon la méthode de T. NAKAYAMA et. coll., Chem. Pharm. Bull., 32, 3968 (1984).

A une solution d'un mélange de 9,84 g d'acide 6-nitro-naphtalène-1-carboxylique et d'acide 3-nitro-naphtalène-1-carboxylique dans 200 cm3 de chloroforme et de 60 cm3 de diméthylformamide, on ajoute 9,9 g de chlorohydrate de l'ester méthylique de la L-méthionine, 6,8 g d'hydroxy-1 benzotriazole, 5 cm3 de triéthylamine et 10,3 g de dicyclohexylcarbodiimide. Le mélange réactionnel est agité pendant 2 jours à une température voisine de 20°C puis filtré sur verre fritté, lavé par 50 cm3 de chloroforme. La solution organique est lavée 2 fois par 200 cm3 d'une solution aqueuse de bicarbonate de sodium à 10 % (p/v) puis par une solution aqueuse d'acide citrique à 10 % (p/v), par de l'eau distillée puis par une solution aqueuse saturée de chlorure de sodium. La phase organique est séchée sur sulfate de magnésium, filtrée puis concentrée à sec sous pression réduite. On obtient 13,3 g d'une huile que l'on purifie par chromatographie sur silice en éluant avec un mélange cyclohexane-acétate d'éthyle (1-1 en volumes). On obtient ainsi 0,64 g de l'ester méthylique de la N-[(6-nitro-naphtyl)-1-carbonyl]-L-méthionine sous forme d'un solide ainsi que 3,7 g d'un mélange de l'ester méthylique de la N-[(6-nitro-naphtyl)-1-carbonyl]-L-méthionine et de l'ester méthylique de la N-[(3-nitro-naphtyl)-1-carbonyl]-L-méthionine.

L'ester méthylique de la N-[(6-nitro-naphtyl)-1-carbonyl]-L-méthionine présente les caractéristiques sont les suivantes :
- spectre de résonance magnétique nucléaire du proton (300 MHz ; (CD₃)₂SO d6 ; δ en ppm) : de 2,10 (mt, 2H : CH₂) ; 2,12 (s, 3H : SCH₃) ; 2,65 (mt, 2H : CH₂S) ; 3,77 (s, 3H : COOCH₃) ; 4,70 (mt, 1H : CHCOO) ; 7,82 (t, J = 7,5 Hz, 1H : H en 3) ; 7,95 et 8,45 (2d, J = 7,5 Hz, 1H chacun : H en 2 et 4) ; 8,36 (dd, J = 9 et 2 Hz, 1H : H en 7) ; 8,48 (d large, J = 9 Hz, 1H : H en 8) ; 9,12 (d, J = 2 Hz, 1H : H en 5) ; 9,18 (d, J = 7,5 Hz, 1H : CONH).

A une solution de 0,64 g de l'ester méthylique de la N-[(6-nitro-naphtyl)-1-carbonyl]-L-méthionine dans 40 cm3 d'éthanol, on ajoute 2 g de dihydrate de chlorure d'étain (II). Le mélange réactionnel est agité pendant 30 minutes à une température voisine de 70°C, puis refroidi à une température voisine de 20°C. On ajoute 40 cm3 d'acétate d'éthyle. Le mélange réactionnel est versé sur de la glace puis amené à pH voisin de 7-8 par addition d'une solution aqueuse d'hydrogénocarbonate de sodium à 5 % (p/v). Le mélange obtenu est filtré sur verre fritté garni de célite. La phase organique est séparée par décantation et la phase aqueuse est extraite 3 fois par 150 cm3 d'acétate d'éthyle. Les phases organiques sont réunies, séchées sur du sulfate de magnésium, filtrées et concentrées à sec sous pression réduite. On obtient ainsi 0,66 g d'une huile que l'on purifie par chromatographie sur silice en éluant avec un mélange cyclohexane-acétate d'éthyle (1-1 en volumes). On obtient ainsi 0,38 g d'ester méthylique de la N-[(6-amino-naphtyl)-1-carbonyl]-L-méthionine sous forme d'une huile dont les caractéristiques sont les suivantes :
- spectre de résonance magnétique nucléaire du proton (200 MHz, (CD₃)₂SO d6 , δ en ppm) : 2,06 (mt, 2H : CH₂) ; 2,09 (s, 3H : SCH₃) ; 2,60 (mt, 2H : CH₂S) ; 3,80 (s, 3H : COOCH₃) ; 4,63 (mt, 1H : CHCOO) ;5,48 (s, 2H : Ar-NH₂) ; 6,86 (dd, J = 9 et 2 Hz, 1H : H en 7) ; de 7,19 à 7,62 (2d, J = 8 Hz, 1H chacun : H en 2 et 4) ; 7,32 (t, J = 8 Hz, 1H : H en 3) ; 7,90 (d large), J = 9 Hz, 1H : H en 8) ; 8,82 (d, J = 7,5 Hz, 1H : CONH).

A une solution de 0,66 g d'ester méthylique de la N-[(6-amino-naphtyl)-1-carbonyl]-L-méthionine dans 20 cm3 de dichlorométhane on ajoute 1,02 g de S-triphénylméthyl-N-tert-butoxycarbonyl-cystéine, 0,297 g de 1-hydroxybenzotriazole et 0,42 g de chlorhydrate de 1-(3-diéthylaminopropyl)-3-éthylcarbodiimide. Le mélange réactionnel est agité pendant 24 heures à une température voisine de 20°C. La solution est lavée 3 fois par 15 cm3 d'eau distillée. La phase organique est séchée sur du sulfate de magnésium, filtrée et concentrée à sec sous pression réduite. On obtient une meringue qui est purifiée par chromatographie sur silice [éluant : dichlorométhane-acétate d'éthyle (8-2 en volumes)]. On obtient 0,6 g de l'ester méthylique de la N-[6-(2(R)-tert-butoxycarbonylamino-3-triphénylméthylthiopropionylamino)-naphtyl-1-carbonyl]-L-méthionine sous forme d'un solide blanc dont les caractéristiques sont les suivantes :
- spectre de résonance magnétique nucléaire du proton (400 MHz, CDCl₃, à une température de 333°K, δ en ppm) : 1,45 (s, 9H : OC(CH₃)₃) ; 2,14 (s, 3H : SCH₃) ; 2,16 et 2,35 (2 mts, 1H chacun : CH₂) ; 2,55 (t, J = 7,5 Hz, 2H : CH₂S) ; 2,73 et 2,83 (2 dd, respectivement J = 13 et 5,5 Hz et J = 13 et 7,5 Hz, 1H chacun : CH₂S) ; 3,83 (s, 3H : COOCH₃) ; 3,99 (mt, 1H : CHN) ; 4,84 (d, J = 7,5 Hz, 1H : NHCOO) ; 5,03 (mt, 1H : CHCOO) ; 6,64 (d, J = 8 Hz, 1H : ArCONH) ; de 7,05 à 7,50 (mt, 16H : SC(C₆H₅)₃ et H 3) ; 7,38 (dd, J = 9 et 1,5 Hz, 1H : H 7) ; 7,59 (d, J = 7,5 Hz, 1H : H 2) ; 7,85 (d large, J = 7,5 Hz, 1H : H 4) ; 8,22 (mf, 1H : ArNHCO) ; 8,27 (d, J = 1,5 Hz, 1H : H 5) ; 8,33 (d, J = 9 Hz, 1H : H 8).

A une solution de 0,3 g d'ester méthylique de la N-[6-(2(R)-tert-butoxycarbonylamino-3-triphénylméthylthio-propionylamino)-naphtyl-1-carbonyl]-L-méthionine dans 0,75 cm3 d'eau distillée et 7,5 cm3 de tétrahydrofuranne, on ajoute 0,057 g de lithine hydratée. La solution est agitée pendant 20 heures à une température voisine de 20°C, puis concentrée sous pression réduite. On obtient 0,29 g de N-[6-(2(R)-tert-butoxycarbonylamino-3-triphénylméthylthio-propionylamino)naphtyl-1-carbonyl]-L-méthionine sous forme d'une meringue. Ce produit est utilisé dans l'étape suivante sans autre purification.

A un mélange de 0,29 g de N-[6-(2(R)-tert-butoxycarbonylamino-3-triphénylméthylthio-propionylamino)-naphtyl-1-carbonyl]-L-méthionine dans 2,6 cm3 de dichlorométhane, on ajoute, à une température voisine de 20°C, 0,1 cm3 de triéthylsilane, puis 2,6 cm3 d'acide trifluoroacétique. Le mélange réactionnel est agité pendant 2 heures à une température voisine de 20°C, puis concentré sous pression réduite. Le résidu est trituré 3 fois par 25 cm3 d'hexane, 3 fois par 25 cm3 de pentane, 3 fois par 25 cm3 d'éther éthylique puis séché sous pression réduite. Le résidu est purifié par chromatographie liquide à haute performances (phase C18) en éluant avec un mélange acétonitrile-eau contenant 0,1 % d'acide trifluoroacétique. On obtient 0,02 g de trifluoroacétate de N-[6-(2(R)-amino-3-mercapto-propionylamino)-naphtyl-1-carbonyl]-L-méthionine sous forme d'une poudre dont les caractéristiques sont les suivantes :
- spectre de résonance magnétique nucléaire du proton (300 MHz, (CD₃)₂SO d6, δ en ppm) : 2,08 (mt, 2H : CH₂) ; 2,11 (s, 3H : SCH₃) ; 2,63 (mt, 2H : CH₂S) ; 3,11 (mt, 2H : CH₂S) ; 4,22 (mt, 1H : CHN) ; 4,61(mt, 1H : CHCOO) ; 7,58 (mt, 2H : H 2 et H 3) ; 7,68 (dd, J = 9 et 2,5 Hz, 1H : H 7) ; 8,01 (dd, J = 7,5 et 2,5 Hz, 1H : H 4) ; 8,28 (d, J = 9 Hz, 1H : H 8) ; 8,38 (d, J = 2,5 Hz, 1H : H 5) ; 8,43 (mf, 3H : NH₃⁺CF₃COO⁻); 8,88 (d, J = 7,5 Hz, 1H : ArCONH) ; 10,82 (s, 1H : ArNHCO).
- analyse élémentaire : C₁₉H₂₃N₃O₄S₂, 1 CF₃CO₂H :

| | | | | |
|---|---|---|---|---|
| Calculé (%) | C = 47,1 ; | H = 4,52 ; | N = 7,85 ; | S = 11,9 |
| Trouvé (%) | C = 47,1 ; | H = 4,3 ; | N = 7,6 ; | S = 10,7. |

### EXEMPLE 18

En opérant comme dans l'exemple 17 pour la préparation du trifluoroacétate de la N-[6-(2(R)-amino-3-mercapto-propionylamino)-naphtyl-1-carbonyl]-L-méthionine, mais à partir de 0,25 g de l'ester méthylique de la N-[6-(2(R)-tert-butoxycarbonylamino-3-triphénylméthylthio-propionylamino)-naphtyl-1-carbonyl]-L-méthionine, on obtient 0,035 g de trifluoroacétate de l'ester méthylique de la N-[6-(2(R)-amino-3-mercapto-propionylamino)-naphtyl-1-carbonyl]-L-méthionine dont les caractéristiques sont les suivantes :
- spectre de résonance magnétique nucléaire du proton (400 MHz, (CD₃)₂SO d6, δ en ppm) : 2,08 (mt, 2H : CH₂) ; 2,10 (s, 3H : SCH₃) ; 2,64 (mt, 2H : CH₂S) ; 3,11 (d, J = 6 Hz, 2H : CH₂S) ; 3,74 (s, 3H : COOCH₃) ; 4,19 (t, J = 6 Hz, 1H : CHN) ; 4,67 (mt, 1H : CHCOO) ; de 7,50 à 7,60 (mt, 2H : H 2 et H 3) ; 7,68 (dd, J = 9 et 1,5 Hz, 1H : H 7) ; 8,00 (d large, J = 8 et 2 Hz, 1H : H 4) ; 8,23 (d, J = 9 Hz, 1H : H 8) ; 8,36 (d, J = 1,5 Hz, 1H : H 5) ; 8,43 (mf, 2H : NH₂) ; 8,98 (d, J = 8 Hz, 1H : ArCONH) ; 10,78 (s, 1H : ArNHCO).
- analyse élémentaire : C₂₀H₂₅N₃O₄S₂, 1 CF₃CO₂H :

| | | | | |
|---|---|---|---|---|
| Calculé (%) | C = 48,08 ; | H = 4,77 ; | N = 7,65 ; | S = 11,6 |
| Trouvé (%) | C = 48,3 ; | H = 4,6 ; | N = 7,5 ; | S = 11,2. |

### EXEMPLE 19

A une solution de 0,34 g d'ester méthylique de la N-[(6-amino-naphtyl)-1-carbonyl]-L-méthionine dans 20 cm3 de méthanol on ajoute 1,34 g de S-triphénylméthyl-N-tert-butoxycarbonyl-cystéinal préparé selon le procédé décrit dans le brevet EP 0618 221 A2, 0,17 cm3 d'acide acétique, du tamis moléculaire (3Å) puis 0,19 g de cyanoborohydrure de sodium. Le mélange réactionnel est agité pendant 3 jours à une température voisine de 20°C puis filtré sur verre fritté garni de célite. Le verre fritté est lavé par du méthanol. Le filtrat est concentré sous pression réduite, redissous dans 100 cm3 d'acétate d'éthyle et lavé par 100 cm3 d'une solution aqueuse d'hydrogénocarbonate de sodium à 10 % (p/v), 80 cm3 d'une solution aqueuse d'acide citrique à 10 % (p/v), 100 cm3 d'eau distillé, encore 100 cm3 d'une solution aqueuse d'hydrogénocarbonate de sodium à 10 % (p/v), et enfin par 100 cm3 d'une solution aqueuse saturée de chlorure de sodium. La phase organique est séchée sur du sulfate de magnésium, filtrée et concentrée à sec sous pression réduite. On obtient une meringue qui est purifiée par chromatographie sur silice [éluant : cyclohexane-acétate d'éthyle (1-3 en volumes)]. On obtient 0,48 g de l'ester méthylique de la N-[6-(2(R)-tert-butoxycarbonylamino-3-triphénylméthylthio-propylamino)-naphtyl-1-carbonyl]-L-méthionine sous forme d'un solide jaune dont les caractéristiques sont les suivantes :
- spectre de résonance magnétique nucléaire du proton (300 MHz, (CD₃)₂SO d6, δ en ppm) : 1,40 (s, 9H : (CH₃)₃) ; 2,10 (mt, 2H : CH₂) ; 2,12 (s, 3H : SCH₃) ; de 2,20 à 2,55 et 2,63 (2 mts, 2H chacun : CH₂S) ; 3,06 (mt, 2H : NCH₂) ; 3,74 (mt, 1H : CHN) ; 3,74 (s, 3H : COOCH₃) ; 4,67 (mt, 1H : CHCOO) ; 5,92 (t large, J = 5,5 Hz, 1H : ArNH) ; 6,75 (s large, 1H : H 5) ; 6,89 et 7,63 (2d, J = 7,5 Hz, 1H chacun : H en 2 et H en 4) ; 6,95 (dd, J = 9 et 1,5 Hz, 1H : H en 7) ; de 7,20 à 7,45 (mt, 16H : H en 3 et H aromatiques) ; 7,93 (d large, J = 9 Hz, 1H : H en 8) ; 8,85 (d, J = 7,5 Hz, 1H : CONH).

A une solution de 0,48 g d'ester méthylique de la N-[6-(2(R)-tert-butoxycarbonylamino-3-triphénylméthylthio-propylamino)naphtyl-1-carbonyl]-L-méthionine dans 2,6 cm3 d'eau distillée et 7,2 cm3 de tétrahydrofuranne, on ajoute 0,05 g de lithine hydratée. La solution est agitée pendant 20 heures à une température voisine de 20°C, puis concentrée sous pression réduite. Le résidu est dissous dans de l'eau distillé puis amené à pH = 3 par addition d'une solution aqueuse d'acide citrique à 10 % (p/v). La phase aqueuse est extraite 3 fois par 50 cm3 d'acétate d'éthyle. Les phases organiques sont réunies, lavées avec une solution aqueuse saturée de chlorure de sodium, séchées sur du sulfate de magnésium, filtrées et concentrées à sec sous pression réduite. On obtient 0,43 g de N-[6-(2(R)-tert-butoxycarbonylamino-3-triphénylméthylthiopropylamino)-naphtyl-1-carbonyl]-L-méthionine sous forme d'une meringue. Ce produit est utilisé dans l'étape suivante sans autre purification.

A un mélange de 0,42 g de N-[6-(2(R)-tert-butoxycarbonylamino-3-triphénylméthylthio-propylamino)-naphtyl-1-carbonyl]-L-méthionine dans 1,0 cm3 d'éthanedithiol, on ajoute, à une température voisine de 20°C, 20 cm3 d'acide trifluoroacétique. Le mélange réactionnel est agité pendant 2 heures à une température voisine de 20°C, puis concentré sous pression réduite. Le résidu est trituré 3 fois par 25 cm3 d'éther éthylique puis séché sous pression réduite. Le résidu est purifié par chromatographie liquide à haute performances (phase C18) en éluant avec un mélange acétonitrile-eau contenant 0,1 % d'acide trifluoroacétique. On obtient 0,14 g de trifluoroacétate de la N-[6-(2(R)-amino-3-mercapto-propylamino)-naphtyl-1-carbonyl]-L-méthionine sous forme d'une poudre dont les caractéristiques sont les suivantes :
- spectre de résonance magnétique nucléaire du proton (300 MHz, (CD₃)₂SO d6, δ en ppm) : 2,05 (mt, 2H : CH₂) ; 2,10 (s, 3H : SCH₃) ; 2,62 et 2,88 (2 mts, 2H chacun: CH₂S) ; de 3,30 à 3,70 (mt, 3H : CHN et NCH₂) ; 4,58 (mt, 1H : CHCOO) ; 6,20 (mf, 1H : ArNH) ; 6.92 (s large, 1H : H en 5) ; 7,05 (dd, J = 9 et 1,5 Hz, 1H : H en 7) ; 7,28 et 7,70 (2d, J = 7,5 Hz, 1H chacun : H en 2 et H en 4) ; 7,40 (t, J = 7,5 Hz, 1H : H en 3) ; 8,08 (d large, J = 9 Hz, 1H : H en 8) ; 8,09 (mf, 3H : NH₃⁺) ; 8,70 (d, J = 7,5 Hz, 1H : CONH)
- analyse élémentaire : C₁₉H₂₅N₃O₃S₂, 1,4 CF₃CO₂H :

| | | | | |
|---|---|---|---|---|
| Calculé (%) | C = 46,16 ; | H = 4,69 ; | N = 7,41 ; | S = 11,31 |
| Trouvé (%) | C = 46,13 ; | H = 4,59 ; | N = 7,45 ; | S = 11,25. |

### EXEMPLE 20

En opérant comme dans l'exemple 19 pour la préparation du trifluoroacétate de la N-[6-(2(R)-amino-3-mercapto-propylamino)-naphtyl-1-carbonyl]-L-méthionine, mais à partir de 0,6 g d'ester méthylique de la N-[6-(2(R)-tert-butoxycarbonylamino-3-triphénylméthylthio-propylamino)-naphtyl-1-carbonyl]-L-méthionine, on obtient 0,15 g de trifluoroacétate de l'ester méthylique de la N-[6-(2(R)-amino-3-mercapto-propylamino)-naphtyl-1-carbonyl]-L-méthionine dont les caractéristiques sont les suivantes :
- spectre de résonance magnétique nucléaire du proton (300 MHz, (CD₃)₂SO d6 avec ajout de quelques gouttes de CD₃COOD d4, δ en ppm) : 2,05 (mt, 2H : CH₂) ; 2,05 (s, 3H : SCH₃) ; 2,56 et 2,83 (2 mts, 2H chacun : CH₂S) ; de 3,35 à 3,60 (mt, 3H : CHN et NCH₂) ; 3,68 (s, 3H : COOCH₃) ; 4,54 (mt, 1H : CHCOO) ; 6.90 (d, J = 1,5 Hz, 1H : H en 5) ; 7,03 (dd, J = 9 et 1,5 Hz, 1H : H en 7) ; 7,28 et 7,67 (2d, J = 7,5 Hz, 1H chacun : H en 2 et H en 4) ; 7,34 (t, J = 7,5 Hz, 1H : H en 3) ; 8,00 (d large, J = 9 Hz, 1H : H en 8).
- analyse élémentaire : C₂₀H₂₇N₃O₃S₂, 1,2 CF₃CO₂H

| | | | | |
|---|---|---|---|---|
| Calculé (%) | C = 48,19 ; | H = 5,09 ; | N = 7,52 ; | S = 11,48 |
| Trouvé (%) | C = 48,05 ; | H = 4,98 ; | N = 7,75 ; | S = 11,80. |

### EXEMPLE 21

A une solution de 0,57 g d'acide méta-chloroperbenzoïque dans 6 cm3 de dichlorométhane à une température voisine de 0°C, on additionne au goutte à goutte pendant une période de 10 minutes, 0,5 g d'ester méthylique de la N-[(6-nitronaphtyl)-1-carbonyl]-L-méthionine dissous dans 4 cm3 de dichlorométhane. Le mélange réactionnel est agité pendant 20 heures à une température voisine de 20°C, puis refroidi à une température voisine de 0°C, on ajoute 5 cm3 d'une solution d'hydroxyde de sodium normale. Après décantation, la phase aqueuse est extraite 2 fois par 10 cm3 de dichlorométhane. Les phases organiques sont réunies, lavées avec une solution aqueuse saturée de chlorure de sodium, séchées sur du sulfate de magnésium, filtrées et concentrées à sec sous pression réduite. On obtient 0,6 g de 2(S)-(6-nitro-1-naphtoylamino)-4-méthylsulfonyl-butanoate de méthyle qui sont utilisés dans l'étape suivante sans autre purification.

A une solution de 0,42 g d'ester méthylique de la 2(S)-(6-nitro-1-naphtoylamino)-4-méthylsulfonyl-butanoate de méthyle dissous dans 30 cm3 d'acétate d'éthyle et 20 cm3 d'éthanol, on additionne 0,04 g de palladium sur charbon à 10 % et on soumet la solution à une pression d'hydrogène de 1,5 atmosphère pendant 3 heures à une température voisine de 20°C. Le mélange réactionnel est filtré sur un verre fritté garni de célite et concentré à sec sous pression réduite. On obtient 0,6 g de 2(S)-(6-amino-1-naphtoylamino)-4-méthylsulfonyl-butanoate de méthyle dont les caractéristiques sont les suivantes :
- spectre de résonance magnétique nucléaire (300 MHz, (CD₃)₂SO d6 avec ajout de quelques gouttes de CD₃COOD d4, δ en ppm) : de 2,10 à 2,40 (mt, 2H : CH₂) ; 3,00 (s, 3H : SO₂CH₃) ; de 3,15 à 3,40 (mt, 2H : CH₂SO₂) ; 3,75 (s, 3H : COOCH₃) ; 4,67 (mt, 1H : CHCOO) ; 6.89 (d, J = 2 Hz, 1H : H 5) ; 7,01 (dd, J = 9 et 2 Hz, 1H : H 7) ; 7,25 (d large, J = 7,5 Hz, 1H : H 2) ; 7,32 (t, J = 7,5 Hz, 1H : H 3) ; 7,63 (d large, J = 7,5 Hz, 1H : H 4) ; 7,95 (d, J = 9 Hz, 1H : H 8) ; 8,85 (d résiduel, J = 7,5 Hz : ArCONH).

En opérant comme dans l'exemple 19 pour la préparation de l'ester méthylique de la N-[6-(2(R)-tert-butoxycarbonylamino-3-triphénylméthylthiopropylamino)-naphtyl-1-carbonyl]-L-méthionine mais à partir de 0,4 g de 2(S)-(6-amino-1-naphtoylamino)-4-méthylsulfonyl-butanoate de méthyle, on obtient 0,55 g de 2(S)-[6-(2(R)-tert-butoxycarbonylamino-3-triphénylméthylthio-propylamino)-1-naphtoylamino]-4-méthylsulfonyl-butanoate de méthyle dont les caractéristiques sont les suivantes :
- spectre de résonance magnétique nucléaire (300 MHz, (CD₃)₂SO d6, δ en ppm) : 1,40 (s, 9H : OC(CH₃)₃) ; de 2,20 à 2,45 (mt, 2H : CH₂) ; de 2,50 à 2,65 (mt : CH₂S) ; 3,03 (s, 3H : SO₂CH₃) ; de 2,95 à 3,40 (mt, 4H : NCH₂ et CH₂SO₂) ; de 3,70 à 3,85 (mt, 1H : CHN) ; 3,77 (s, 3H : COOCH₃) ; 4,68 (mt, 1H : CHCOO) ; 5,92 (t large, J = 5 Hz, 1H : ArNH) ; 6.75 (s large, 1H : H 5) ; 6,90 (d, J = 8 Hz, 1H : NHCOO) ; 6,94 (d large, J = 9 Hz, 1H : H 7) ; de 7,25 à 7,45 (mt, 17H : SC(C₆H₅)₃ - H 2 et H 3) ; 7,65 (d large, J = 7,5 Hz, 1H : H 4) ; 7,95 (d, J = 9 Hz, 1H : H 8) ; 8,94 (d, J = 7,5 Hz, 1H : ArCONH).

En opérant comme dans l'exemple 19 pour la préparation de la N-[6-(2(R)-tert-butoxycarbonylamino-3-triphénylméthylthio-propylamino)-naphtyl-1-carbonyl]-L-méthionine, mais à partir de 0,36 g de 2(S)-[6-(2(R)-tert-butoxycarbonylamino-3-triphénylméthylthio-propylamino)-1-naphtoylamino]-4-méthylsulfonyl-butanoate de méthyle, on obtient 0,35 g d'acide 2(S)-[6-(2(R)-tert-butoxycarbonylamino-3-triphénylméthylthiopropylamino)-1-naphtoylamino]-4-méthylsulfonyl-butanoïque qui sont utilisés dans l'étape suivante sans autre purification.

En opérant comme dans l'exemple 19 pour la préparation du trifluoroacétate de la N-[6-(2(R)-amino-3-mercapto-propylamino)-naphtyl-1-carbonyl]-L-méthionine mais à partir de 0,35 g d'acide 2(S)-[6-(2(R)-tert-butoxycarbonylamino-3-triphénylméthylthiopropylamino)-1-naphtoylamino]-4-méthylsulfonylbutanoïque, on obtient 0,132 g de trifluoroacétate de l'acide 2(S)-[6-(2(R)-amino-3-mercapto-propylamino)-1-naphtoylamino]-4-méthylsulfonyl-butanoïque dont les caractéristiques sont les suivantes :
- spectre de résonance magnétique nucléaire (300 MHz, (CD₃)₂SO d6, δ en ppm) : de 2,10 à 2,45 (mt, 2H : CH₂) ; 2,90 (mt, 2H : CH₂S) ; 3,04 (s, 3H : SO₂CH₃) ; de 3,15 à 3,70 (mt : CH₂SO₂ et NCH₂CHN) ; 4,60 (mt, 1H : CHCOO) ; 6,24 (mf, 1H : ArNH) ; 6.94 (d, J = 2 Hz, 1H : H 5) ; 7,08 (dd, J = 9 et 2 Hz, 1H : H 7) ; 7,35 (d large, J = 7,5 Hz, 1H : H 2) ; 7,42 (t, J = 7,5 Hz, 1H : H 3) ; 7,72 (d large, J = 7,5 Hz, 1H : H 4) ; 8,05 (d, J = 9 Hz, 1H : H 8) ; 8,08 (mf, 3H : NH₃⁺CF₃COO⁻); 8,83 (d, J = 7,5 Hz, 1H : ArCONH).
- analyse élémentaire : C₁₉H₂₅N₃O₅S₂, 1,6 CF₃CO₂H :

| | | | | |
|---|---|---|---|---|
| Calculé (%) | C = 42,87 ; | H = 4,31 ; | N = 6,76 ; | S = 10,31 |
| Trouvé (%) | C = 42,4 ; | H = 4,2 ; | N = 6,9 ; | S = 10,4 |

### EXEMPLE 22

En opérant comme dans l'exemple 19 pour la préparation du trifluoroacétate de la N-[6-(2(R)-amino-3-mercapto-propylamino)-naphtyl-1-carbonyl]-L-méthionine, mais à partir de 0,16 g de 2(S)-[6-(2(R)-tert-butoxycarbonylamino-3-triphénylméthylthio-propylamino)-1-naphtoylamino]-4-méthylsulfonyl-butanoate de méthyle, on obtient 0,035 g de trifluoroacétate du 2(S)-[6-(2(R)-amino-3-mercaptopropylamino)-1-naphtoylamino]-4-méthylsulfonyl-butanoate de méthyle dont les caractéristiques sont les suivantes :
- spectre de résonance magnétique nucléaire (300 MHz, (CD₃)₂SO d6, δ en ppm) : de 2,25 à 2,40 (mt, 2H : CH₂) ; 2,91 (mt, 2H : CH₂S) ; 3,04 (s, 3H : SO₂CH₃) ; de 3,20 à 3,65 (mt : CH₂SO₂ et NCH₂CHN) ; 3,77 (s, 3H : COOCH₃) ; 4,67 (mt, 1H : CHCOO) ; 6,24 (mf, 1H : ArNH) ; 6.94 (d, J = 2 Hz, 1H : H 5) ; 7,08 (dd, J = 9 et 2 Hz, 1H : H 7) ; 7,35 (d large, J = 7,5 Hz, 1H : H 2) ; 7,42 (t, = 7,5 Hz, 1H : H 3) ; 7,65 (d large, J = 7,5 Hz, 1H : H4) ; 8,05(d, J = 9 Hz, 1H : H8) ; 8,08 (mf, 3H : NH₃⁺CF₃COO⁻); 8,95 (d, J = 7,5 Hz, 1H : ArCONH)
- analyse élémentaire : C₂₀H₂₇N₃O₅S₂, 1,25 CF₃CO₂H :

| | | | | |
|---|---|---|---|---|
| Calculé (%) | C = 45,34 ; | H = 4,78 ; | N = 7,05 ; | S = 10,76 |
| Trouvé (%) | C = 44,4 ; | H = 4,4 ; | N = 7,0 ; | S = 10,7 |

### EXEMPLE 23

En opérant comme dans l'exemple 17 pour la préparation de l'ester méthylique de la N-[6-(2(R)-tert-butoxycarbonylamino-3-triphénylméthylthiopropionylamino)-naphtyl-1-carbonyl]-L-méthionine mais à partir de 0,66 g d'ester méthylique de la N-[(6-aminonaphtyl)-1-carbonyl]-L-méthionine et de 0,48 g de N,N'-di-tert-butoxycarbonylcystine, on obtient 0,7 g de diester méthylique de la di-{N-[6-(2(R)-tert-butoxycarbonylamino-3-sulfanyl-propionylamino)-naphtyl-1-carbonyl]-L-méthionine} qui sont utilisés dans l'étape suivante sans autre purification.

En opérant comme dans l'exemple 17 pour la préparation du trifluoroacétate de la N-[6-(2(R)-amino-3-mercaptopropionylamino)-naphtyl-1-carbonyl]-L-méthionine, mais à partir de 0,3 g de diester méthylique de la di-{N-[6-(2(R)-tert-butoxycarbonylamino-3-sulfanyl-propionylamino)-naphtyl-1-carbonyl]-L-méthionine}, on obtient 0,06 g de trifluoroacétate du diester méthylique de la di-{N-[6-(2(R)-amino-3-sulfanyl-propionylamino)-naphtyl-1-carbonyl]-L-méthionine} dont les caractéristiques sont les suivantes :
- spectre de résonance magnétique nucléaire du proton (400 MHz, (CD₃)₂SO d6, δ en ppm) : de 2,00 à 2,15 (mt, 2H : CH₂) ; 2,08 (s, 3H : SCH₃) ; 2,62 (mt, 2H : CH₂S) ; 3,22 et 3,44 (respectivement dd et mt, J = 14 et 8 Hz, 1H chacun : CH₂S) ; 3,72 (s, 3H : COOCH₃) ; 4,32 (mt, 1H : CHN) ; 4,67 (mt, 1H : CHCOO) ; de 7,50 à 7,60 (mt, 2H : H 2 et H 3) ; 7,68 (dd, J = 9 et 2 Hz, 1H : H 7) ; 7,97 (d large, J = 8,5 Hz, 1H : H 4) ; 8,24 (d, J = 9 Hz, 1H : H 8) ; 8,35 (d, J = 2 Hz, 1H : H 5) ; 8,53 (mf, 2H : NH₃⁺CF₃COO⁻); 8,98 (d, J = 8 Hz, 1H : ArCONH) ; 10,92 (s, 1H : ArNHCO)
- analyse élémentaire : C₄₀H₄₈N₆O₈S₄, 2 CF₃CO₂H, dihydrate :

| | | | | |
|---|---|---|---|---|
| Calculé (%) | C = 46,6; | H = 4,75; | N = 7,4; | S = 11,3 |
| Trouvé (%) | C = 46,6 ; | H = 4,5 ; | N = 7,1 ; | S = 11,0 |

L'activité inhibitrice de la farnésyltransférase et de la farnésylation de la protéine Ras peut être mise en évidence dans le test suivant :

L'activité farnésyltransférase est déterminée par la quantité de (³H) farnésyl transférée à partir du (³H) farnésylpyrophosphate [(³H) FPP) sur la protéine p21 H-Ras. Le mélange réactionnel standard est composé, pour un volume final de 60 µl, de Tris-HCl 50mM, MgCl₂ 5mM, dithiotréitol 5 mM, octyl-β-D-glucopyranoside 0,2 %, p21 H-ras 200 picomoles, (³H) FPP (à 61000 dpm/picomole) 4,5 picomoles.

La réaction est initiée par addition d'environ 5 ng de farnésyltransférase humaine purifiée à partir de cultures de cellules THP1. Après incubation pendant 20 minutes à 37°C en plaque de microtitration contenant 96 trous de 1 cm3 par plaque (Titer Plate®, Beckman), la réaction est stoppée par addition de 0,4 cm3 de SDS à 0,1 % dans le méthanol à 0°C. Le mélange est ensuite additionné de 0,4 cm3 d'acide trichloroacétique (TCA) à 30 % dans le méthanol. Les plaques sont laissées pendant 1 heure dans la glace. Le contenu précipité est alors retenu sur membrane en fibre de verre Filtermat®, Pharmacia) avec l'unité de filtration (Combi Cell Harvester®, Skatron) et rincé avec de l'acide trichloroacétique à 6 % dans l'eau distillée. Les membranes sont séchées au four à micro-ondes puis imprégnées de scintillant par fusion sous air chaud de Meltilex® (Pharmacia) et enfin comptées en cpm dans un compteur β-Plate® (LKB). Chaque essai est répété 3 fois.

L'unité d'activité est définie par 1 picomole de (³H) FPP transférée sur p21 H-Ras en 20 minutes.

Les pourcentages d'inhibition sont obtenus par comparaison des essais avec et sans inhibiteur après déduction des blancs, les IC₅₀ étant mesurées à partir des inhibitions obtenues avec 9 concentrations différentes en utilisant les logiciels Enzfitter® ou Grafit®.

L'activité sur cellules peut être déterminée de la manière suivante :

La lignée cellulaire est la lignée THAC (cellules CCL 39 transfectées par Ha-Ras activé) selon K. Seuwen et coll., EMBO J., 7(1) 161-168 (1988). Les cellules sont ensemencées dans des boîtes de Petri de 6 cm de diamètre contenant un milieu DMEM, sérum de veau foetal 5 %, G418 1 %.

Après 24 heures de culture, le milieu de culture est changé (avec ou sans sérum) et le produit à étudier est ajouté en solution dans le diméthylformamide (DMF), en présence ou en absence de DTT (concentrations finales de 0,5 % en DMF et 0,1mM en DTT). Après 24 heures de culture à 37°C, les cellules sont lysées dans 1 cm3 de tampon de lyse (Tris, HCI 20mM, Triton X114 1 %, MgCl₂ 5 mM, DIT 7 mM, NaCl 150 mM, pH = 7,4). Les lysats sont clarifiés par centrifugation à 4.000 tours/minute pendant 10 minutes. L'extraction par le Triton X114 permet de séparer la protéine Ras farnésylée de la protéine Ras non farnésylée [C. BORDIER, J. Biol. Chem., 256 (4), 1604-1607 (1981)]. La protéine Ras farnésylée qui est plus hydrophobe se trouve dans la phase détergente tandis que la protéine Ras non farnésylée est dans la phase aqueuse. Les échantillons sont dénaturés par chauffage à 95°C dans du tampon de dénaturation pour électrophorèse et déposées sur un gel de polyacrylamide à 14 %. Lorsque le colorant atteint le bas du gel, les protéines du gel sont transférées sur une membrane PVDF. La protéine Ras est révélée par la technique de Western blot : la membrane est incubée avec un anticorps monoclonal spécifique anti-Ras (pan-Ras Ab3, Oncogène Science) puis avec de la protéine A marquée à ¹²⁵I. Après autoradiographie, les bandes sont identifiées, découpées et comptées dans un compteur γ. La radioactivité des bandes correspondant à Ras farnésylée et à Ras non farnésylée permet de déterminer le pourcentage d'inhibition de la farnésylation de la protéine Ras.

Les résultats obtenus sont rassemblés dans le tableau I.

**TABLEAU I**

| PRODUIT | Activité inhibitrice IC₅₀ | % d'inhibition sur cellules (THAC) |
|---|---|---|
| Exemple 1 | 48 | |
| Exemple 3 | 5,6 | |
| Exemple 5 | 69 | |
| Exemple 8 | 100 | |
| Exemple 9 | 70 | |
| Exemple 10 | 21 | |
| Exemple 19 | 1,8 | 31 à 50 µM |
| Exemple 20 | 80 | 50 à 10 µM |
| Exemple 21 | 4 nM | |

Les nouveaux produits de formule générale (I) peuvent se présenter sous forme de sels non toxiques et pharmaceutiquement acceptables. Ces sels non toxiques comprennent les sels avec les acides minéraux (acides chlorhydrique, sulfurique, bromhydrique, phosphorique, nitrique) ou avec les acides organiques (acides acétique, trifluoroacétique, propionique, succinique, maléique, hydroxymaléique, benzoïque, fumarique, méthanesulfonique ou oxalique) ou avec les bases minérales (soude, potasse, lithine, chaux) ou organiques (amines tertiaires comme la triéthylamine, la pipéridine, la benzylamine) selon la nature des symboles R₁ et R₂ du produit de formule générale (I).

La présente invention concerne également les compositions pharmaceutiques qui contiennent au moins un produit de formule générale (I) en association avec un ou plusieurs diluants ou adjuvants pharmaceutiquement acceptables qu'ils soient inertes ou physiologiquement actifs.

Ces compositions peuvent être administrées par voie orale, parentérale ou rectale.

Les compositions pour administration orale comprennent des comprimés, des pilules, des poudres ou des granulés. Dans ces compositions le produit actif selon l'invention est mélangé à un ou plusieurs diluants inertes tels que saccharose, lactose ou amidon. Ces compositions peuvent comprendre des substances autres que les diluants, par exemple un lubrifiant tel que le stéarate de magnésium.

Comme compositions liquides pour administration orale peuvent être utilisées des émulsions pharmaceutiquement acceptables, des solutions, des suspensions, des sirops, des élixirs contenant des diluants inertes tel que l'eau ou l'huile de paraffine. Ces compositions peuvent également comprendre des substances autres que les diluants, par exemple des produits mouillants, édulcorants ou aromatisants.

Les compositions selon l'invention pour administration parentérale peuvent être des solutions stériles aqueuses ou non aqueuses, des suspensions ou des émulsions. Comme solvant ou véhicule, on peut employer le propylèneglycol, un polyéthylèneglycol, des huiles végétales, en particulier l'huile d'olive ou des esters organiques injectables par exemple l'oléate d'éthyle. Ces compositions peuvent également contenir des adjuvants, en particulier des agents mouillants, émulsifiants et dispersants. La stérilisation peut se faire de plusieurs façons, par exemple à l'aide d'un filtre bactériologique, en incorporant à la composition des agents stérilisants ou par chauffage. Elles peuvent être également préparées sous forme de compositions solides stériles qui peuvent être dissoutes au moment de l'emploi dans de l'eau stérile ou tout autre milieu stérile injectable.

Les compositions pour administration rectale sont des suppositoires qui peuvent contenir, outre le produit actif, des excipients tels que le beurre de cacao.

Les compositions selon l'invention sont particulièrement utiles en thérapeutique humaine dans le traitement de cancers d'origines diverses.

En thérapeutique humaine, les doses dépendent de l'effet recherché, de la durée du traitement et des facteurs propres au sujet à traiter.

Généralement, les doses sont comprises, chez l'homme, entre 0,1 et 20 mg/kg par jour par voie intra-péritonéale.

L'exemple suivant illustre une composition selon l'invention.

### EXEMPLE

On dissout 200 mg du produit obtenu à l'exemple 1 dans 100 cm3 de sérum physiologique. La solution obtenue est répartie aseptiquement en ampoules de 10 cm3.

Les ampoules sont administrées en injection unique ou en perfusion.

## Revendications

1. Produits de formule générale : 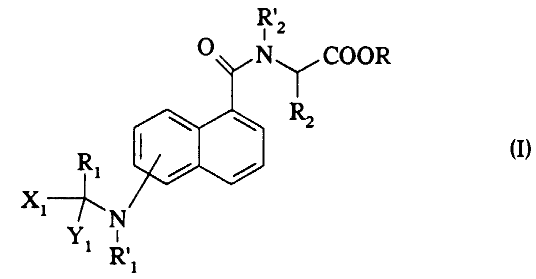 dans laquelle :
R₁ représente un radical de formule générale Y-S-A₁- dans lequel Y représente un atome d'hydrogène, ou un reste d'aminoacide ou un reste d'acide gras ou un radical alkyle ou alkoxycarbonyle ou un radical R₄-S- dans lequel R₄ représente un radical alkyle contenant 1 à 4 atomes de carbone éventuellement substitué par un radical phényle ou un radical de formule générale 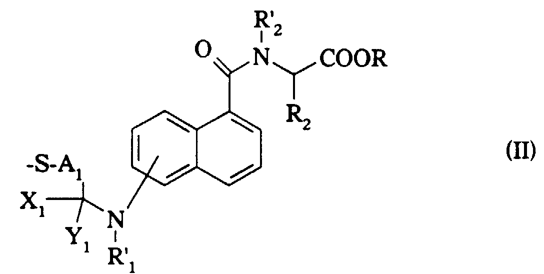 dans laquelle A₁, X₁, Y₁, R'₁, R₂, R'₂ et R sont définis comme ci-après, et A₁ représente un radical alcoylène droit ou ramifié contenant 1 à 4 atomes de carbone éventuellement substitué en a du groupement >C(X₁)(Y₁) par un radical amino, alkylamino contenant 1 à 6 atomes de carbone en chaîne droite ou ramifiée, dialkylamino dont chaque partie alkyle contient 1 à 6 atomes de carbone en chaîne droite ou ramifiée, alcanoylamino contenant 1 à 6 atomes de carbone en chaîne droite ou ramifiée ou alkoxycarbonylamino dont la partie alkyle contient 1 à 6 atomes de carbone en chaîne droite ou ramifiée,
X₁ et Y₁ représentent chacun un atome d'hydrogène ou forment ensemble avec l'atome de carbone auquel ils sont liés un groupement >C=O,
R'₁ représente un atome d'hydrogène ou un radical méthyle,
R₂ représente un radical alkyle, alkényle ou alkynyle droit ou ramifié contenant 1 à 6 atomes de carbone éventuellement substitué par un radical hydroxy, alkoxy contenant 1 à 4 atomes de carbone, mercapto, alkylthio contenant 1 à 4 atomes de carbone, alkylsulfinyle contenant 1 à 4 atomes de carbone ou alkylsulfonyle contenant 1 à 4 atomes de carbone, étant entendu que, lorsque R₂ représente un radical alkyle substitué par un radical hydroxy, R₂ peut former avec la radical carboxy en α une lactone,
R'₂ représente un atome d'hydrogène ou un radical méthyle, et
R représente un atome d'hydrogène ou un radical alkyle contenant 1 à 6 atomes de carbone éventuellement substitué par un radical alkoxy contenant 1 à 4 atomes de carbone, alcoylthio contenant 1 à 4 atomes de carbone, alkylsulfinyle contenant 1 à 4 atomes de carbone, alkylsulfonyle contenant 1 à 4 atomes de carbone, phényle, phénoxy, phénylthio, phénylsulfinyle, phénylsulfonyle, alkylamino contenant 1 à 4 atomes de carbone, dialkylamino dont chaque partie alcoyle contient 1 à 4 atomes de carbone, ou un radical phényle éventuellement substitué par un ou plusieurs atomes ou radicaux choisis parmi les atomes d'halogène et les radicaux alkyles, alkyloxy, alkylthio ou alcanoyle,
étant entendu que le radical 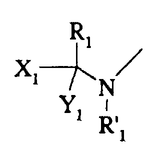 est en position -5 ou -6 du noyau naphtyle.

2. Produits selon la revendication 1 pour lesquels :
R₁ représente un radical de formule Y-S-A₁- dans lequel Y représente un atome d'hydrogène ou un reste lysine ou un reste d'acide gras contenant jusqu'à 20 atomes de carbone et A₁ représente un radical éthylène ou propylène éventuellement substitué par un radical amino ou alkylamino contenant 1 à 4 atomes de carbone
X₁ et Y₁ représentent chacun un atome d'hydrogène ou forment ensemble avec l'atome de carbone auquel ils sont liés un groupement >C=O,
R'₁ représente un atome d'hydrogène ou un radical méthyle,
R₂ représente un radical alkyle contenant 1 à 4 atomes de carbone éventuellement substitué par un radical hydroxy, méthoxy, mercapto, méthylthio, méthylsulfinyle ou méthylsulfonyle,
R'₂ représente un atome d'hydrogène ou un radical méthyle, et
R représente un atome d'hydrogène ou un radical alcoyle contenant 1 à 4 atomes de carbone, éventuellement substitué par un radical alcoxy, ou un radical phényle.

3. Produits selon la revendication 1 pour lesquels :
R₁ représente un radical de formule Y-S-A₁- dans lequel Y représente un atome d'hydrogène et A₁ représente un radical éthylène ou propylène éventuellement substitué par un radical amino,
X₁ et Y₁ représentent chacun un atome d'hydrogène ou forment ensemble avec l'atome de carbone auquel ils sont liés un groupement >C=O,
R'₁ représente un atome d'hydrogène,
R₂ représente un radical méthyle, éthyle, propyle ou butyle éventuellement substitué par un radical hydroxy, méthoxy, mercapto ou méthylthio,
R'₂ représente un atome d'hydrogène, et
R représente un atome d'hydrogène ou un radical alcoyle contenant 1 à 4 atomes de carbone.

4. Produits selon la revendication 1 pour lesquels R₁ représente un radical 2-mercapto éthyle ou 1-amino-2-mercapto éthyle, X₁ et Y₁ représentent chacun un atome d'hydrogène ou forment ensemble avec l'atome de carbone auquel ils sont liés un groupement >C=O, R'₁ représente un atome d'hydrogène, R₂ représente un radical n.butyle ou 2-méthylthio éthyle et R'₂ représente un atome d'hydrogène, et R représente un atome d'hydrogène ou un radical méthyle.

5. Composition pharmaceutique caractérisée en ce qu'elle contient une quantité suffisante d'un produit selon l'une des revendications 1 à 4 en association avec un ou plusieurs diluants ou adjuvants pharmaceutiquement acceptables inertes ou physiologiquement actifs.

## Patentansprüche

1. Produkte der allgemeinen Formel (I) 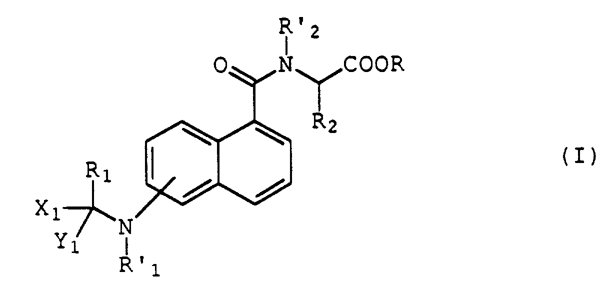 in der
R₁ einen Rest der allgemeinen Formel Y-S-A₁- darstellt, worin Y ein Wasserstoffatom oder den Rest einer Aminosäure oder den Rest einer Fettsäure oder einen Rest Alkyl oder Alkoxycarbonyl oder einen Rest R₄-S- bedeutet, worin R₄ ein Rest Alkyl mit 1 bis 4 Kohlenstoffatomen ist, gegebenenfalls substituiert durch einen Rest Phenyl oder einen Rest der allgemeinen Formel (II) 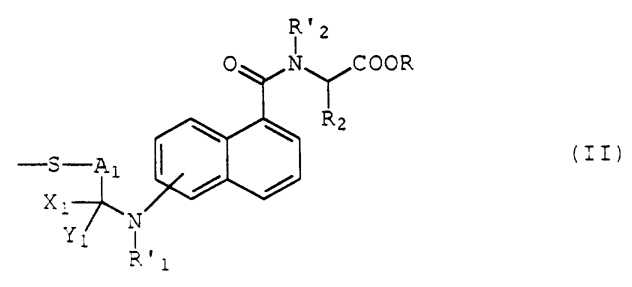 in der A₁, X₁, Y₁, R'₁, R₂, R'₂ und R wie unten definiert sind, und A₁ einen geraden oder verzweigten Rest Alkylen mit 1 bis 4 Kohlenstoffatomen darstellt, gegebenenfalls substituiert in α der Gruppe >C(X₁)(Y₁) durch einen Rest Amino, Alkylamino mit 1 bis 6 Kohlenstoffatomen in gerader oder verzweigter Kette, Dialkylamino, von dem jeder Alkylteil 1 bis 6 Kohlenstoffatome in gerader oder verzweigter Kette enthält, Alkanoylamino mit 1 bis 6 Kohlenstoffatomen in gerader oder verzweigter Kette oder Alkoxycarbonylamino, von dem der Alkylteil 1 bis 6 Kohlenstoffatome in gerader oder verzweigter Kette enthält,
X₁ und Y₁ jeweils ein Wasserstoffatom darstellen oder zusammen mit dem Kohlenstoffatom, an das sie gebunden sind, eine Gruppe >C=O bilden,
R'₁ ein Wasserstoffatom oder ein Rest Methyl ist,
R₂ einen geraden oder verzweigten Rest Alkyl, Alkenyl oder Alkinyl mit 1 bis 6 Kohlenstoffatomen bedeutet, gegebenenfalls substituiert durch einen Rest Hydroxy, Alkoxy mit 1 bis 4 Kohlenstoffatomen, Mercapto, Alkylthio mit 1 bis 4 Kohlenstoffatomen, Alkylsulfinyl mit 1 bis 4 Kohlenstoffatomen oder Alkylsulfonyl mit 1 bis 4 Kohlenstoffatomen, mit der Maßgabe, daß in dem Fall, wo R₂ einen durch einen Rest Hydroxy substituierten Rest Alkyl darstellt, R₂ mit dem Rest Carboxy in α ein Lacton bilden kann,
R'₂ ein Wasserstoffatom oder ein Rest Methyl ist, und
R ein Wasserstoffatom oder einen Rest Alkyl mit 1 bis 6 Kohlenstoffatomen, gegebenenfalls substituiert durch einen Rest Alkoxy mit 1 bis 4 Kohlenstoffatomen, Alkylthio mit 1 bis 4 Kohlenstoffatomen, Alkylsulfinyl mit 1 bis 4 Kohlenstoffatomen, Alkylsulfonyl mit 1 bis 4 Kohlenstoffatomen, Phenyl, Phenoxy, Phenylthio, Phenylsulfinyl, Phenylsulfonyl, Alkylamino mit 1 bis 4 Kohlenstoffatomen, Dialkylamino, von dem jeder Alkylteil 1 bis 4 Kohlenstoffatome enthält, darstellt, oder einen Rest Phenyl bedeutet, gegebenenfalls substituiert durch ein oder mehrere Atome oder Reste, ausgewählt unter den Halogenatomen und den Resten Alkyl, Alkyloxy, Alkylthio oder Alkanoyl,
mit der Maßgabe, daß sich der Rest 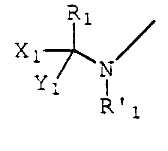 in Position -5 oder -6 des Naphthylkernes befindet.

2. Produkte nach Anspruch 1, worin
R₁ einen Rest der Formel Y-S-A₁- darstellt, in der Y ein Wasserstoffatom oder einen Rest Lysin oder den Rest einer Fettsäure mit bis zu 20 Kohlenstoffatomen bedeutet und A₁ ein Rest Ethylen oder Propylen ist, gegebenenfalls substituiert durch einen Rest Amino oder Alkylamino mit 1 bis 4 Kohlenstoffatomen,
X₁ und Y₁ jeweils ein Wasserstoffatom darstellen oder zusammen mit dem Kohlenstoffatom, an das sie gebunden sind, eine Gruppe >C=O bilden,
R'₁ ein Wasserstoffatom oder ein Rest Methyl ist,
R₂ einen Rest Alkyl mit 1 bis 4 Kohlenstoffatomen bedeutet, gegebenenfalls substituiert durch einen Rest Hydroxy, Methoxy, Mercapto, Methylthio, Methylsulfinyl oder Methylsulfonyl,
R'₂ ein Wasserstoffatom oder ein Rest Methyl ist, und
R ein Wasserstoffatom oder einen Rest Alkyl mit 1 bis 4 Kohlenstoffatomen, gegebenenfalls substituiert durch einen Rest Alkoxy, oder einen Rest Phenyl darstellt.

3. Produkte nach Anspruch 1, worin
R₁ einen Rest der Formel Y-S-A₁- darstellt, in der Y ein Wasserstoffatom bedeutet und A₁ ein Rest Ethylen oder Propylen ist, gegebenenfalls substituiert durch einen Rest Amino,
X₁ und Y₁ jeweils ein Wasserstoffatom darstellen oder zusammen mit dem Kohlenstoffatom, an das sie gebunden sind, eine Gruppe >C=O bilden,
R'₁ ein Wasserstoffatom ist,
R₂ einen Rest Methyl, Ethyl, Propyl oder Butyl bedeutet, gegebenenfalls substituiert durch einen Rest Hydroxy, Methoxy, Mercapto oder Methylthio,
R'₂ ein Wasserstoffatom ist, und
R ein Wasserstoffatom oder einen Rest Alkyl mit 1 bis 4 Kohlenstoffatomen darstellt.

4. Produkte nach Anspruch 1, worin
R₁ einen Rest 2-Mercapto-ethyl oder 1-Amino-2-mercapto-ethyl bedeutet,
X₁ und Y₁ jeweils ein Wasserstoffatom darstellen oder zusammen mit dem Kohlenstoffatom, an das sie gebunden sind, eine Gruppe >C=O bilden,
R'₁ ein Wasserstoffatom ist,
R₂ einen Rest n-Butyl oder 2-Methylthio-ethyl bedeutet,
R'₂ ein Wasserstoffatom ist, und
R ein Wasserstoffatom oder einen Rest Methyl darstellt.

5. Pharmazeutische Zusammensetzung, dadurch gekennzeichnet, daß sie eine ausreichende Menge eines Produktes nach einem der Ansprüche 1 bis 4 in Assoziation mit einem oder mehreren pharmazeutisch akzeptablen, inerten oder physiologisch aktiven Verdünnungsmitteln oder Zusatzstoffen enthält.

## Claims

1. Products of general formula: 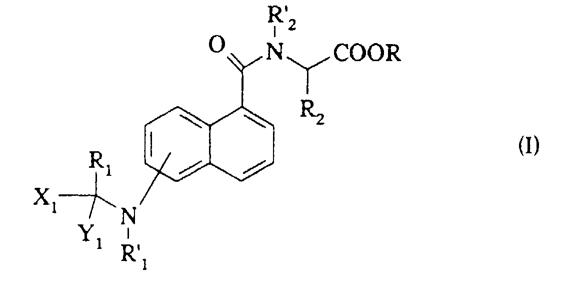 in which:
R₁ represents a radical of general formula Y-S-A₁- in which Y represents a hydrogen atom or an amino acid residue or a fatty acid residue or an alkyl or alkoxycarbonyl radical or an R₄-S- radical in which R₄ represents an alkyl radical containing 1 to 4 carbon atoms, optionally substituted by a phenyl radical, or a radical of general formula: 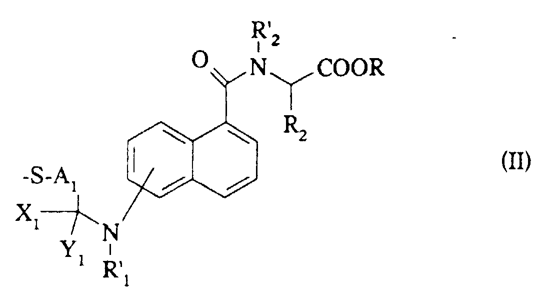 in which A₁, X₁, Y₁, R'₁, R₂, R'₂ and R are defined as below, and A₁ represents a straight or branched alkylene radical containing 1 to 4 carbon atoms, optionally substituted at the position α to the >C(X₁)(Y₁) group by an amino radical, an alkylamino radical containing 1 to 6 straight- or branched-chain carbon atoms, a dialkylamino radical in which each alkyl part contains 1 to 6 straight- or branched-chain carbon atoms, an alkanoylamino radical containing 1 to 6 straight- or branched-chain carbon atoms or an alkoxycarbonylamino radical in which the alkyl part contains 1 to 6 straight- or branched-chain carbon atoms,
X₁ and Y₁ each represent a hydrogen atom or form, together with the carbon atom to which they are bonded, a >C=O group,
R'₁ represents a hydrogen atom or a methyl radical,
R₂ represents a straight or branched alkyl, alkenyl or alkynyl radical containing 1 to 6 carbon atoms, optionally substituted by a hydroxyl radical, an alkoxy radical containing 1 to 4 carbon atoms, a mercapto radical, an alkylthio radical containing 1 to 4 carbon atoms, an alkylsulphinyl radical containing 1 to 4 carbon atoms or an alkylsulphonyl radical containing 1 to 4 carbon atoms, it being understood that, when R₂ represents an alkyl radical substituted by a hydroxyl radical, R₂ can form a lactone with the carboxyl radical at the α position,
R'₂ represents a hydrogen atom or a methyl radical, and
R represents a hydrogen atom or an alkyl radical containing 1 to 6 carbon atoms, optionally substituted by an alkoxy radical containing 1 to 4 carbon atoms, an alkylthio radical containing 1 to 4 carbon atoms, an alkylsulphinyl radical containing 1 to 4 carbon atoms, an alkylsulphonyl radical containing 1 to 4 carbon atoms, a phenyl radical, a phenoxy radical, a phenylthio radical, a phenylsulphinyl radical, a phenylsulphonyl radical, an alkylamino radical containing 1 to 4 carbon atoms or a dialkylamino radical in which each alkyl part contains 1 to 4 carbon atoms, or a phenyl radical, optionally substituted by one or a number of atoms or radicals chosen from halogen atoms and alkyl, alkyloxy, alkylthio or alkanoyl radicals,
it being understood that the radical 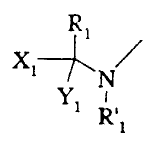 is in the 5- or 6-position of the naphthyl ring.

2. Products according to claim 1 in which:
R₁ represents a radical of formula Y-S-A₁- in which Y represents a hydrogen atom or a lysine residue or a fatty acid residue containing up to 20 carbon atoms and A₁ represents an ethylene or propylene radical optionally substituted by an amino radical or an alkylamino radical containing 1 to 4 carbon atoms,
X₁ and Y₁ each represent a hydrogen atom or form, together with the carbon atom to which they are bonded, a >C=O group,
R'₁ represents a hydrogen atom or a methyl radical,
R₂ represents an alkyl radical containing 1 to 4 carbon atoms, optionally substituted by a hydroxyl, methoxy, mercapto, methylthio, methylsulphinyl or methylsulphonyl radical,
R'₂ represents a hydrogen atom or a methyl radical, and
R represents a hydrogen atom or an alkyl radical containing 1 to 4 carbon atoms, optionally substituted by an alkoxy radical, or a phenyl radical.

3. Products according to claim 1 in which:
R₁ represents a radical of formula Y-S-A₁- in which Y represents a hydrogen atom and A₁ represents an ethylene or propylene radical optionally substituted by an amino radical,
X₁ and Y₁ each represent a hydrogen atom or form, together with the carbon atom to which they are bonded, a >C=O group,
R'₁ represents a hydrogen atom,
R₂ represents a methyl, ethyl, propyl or butyl radical optionally substituted by a hydroxyl, methoxy, mercapto or methylthio radical,
R'₂ represents a hydrogen atom, and
R represents a hydrogen atom or an alkyl radical containing 1 to 4 carbon atoms.

4. Products according to claim 1 in which R₁ represents a 2-mercaptoethyl or 1-amino-2-mercaptoethyl radical, X₁ and Y₁ each represent a hydrogen atom or form, together with the carbon atom to which they are bonded, a >C=O group, R'₁ represents a hydrogen atom, R₂ represents an n-butyl or 2-(methylthio)ethyl radical and R'₂ represents a hydrogen atom, and R represents a hydrogen atom or a methyl radical.

5. Pharmaceutical composition, characterized in that it contains a sufficient amount of a product according to one of claims 1 to 4 in combination with one or a number of inert or physiologically active pharmaceutically acceptable diluents or adjuvants.
